(19) **Europäisches Patentamt**
**European Patent Office**
**Office européen des brevets**

(11) **EP 2 817 615 B1**

(12) **FASCICULE DE BREVET EUROPEEN**

(45) Date de publication et mention de la délivrance du brevet:
**02.05.2018 Bulletin 2018/18**

(21) Numéro de dépôt: **13710496.4**

(22) Date de dépôt: **21.02.2013**

(51) Int Cl.:
***G01N 29/024*** *(2006.01)*     ***G01N 29/07*** *(2006.01)*
***G01N 33/38*** *(2006.01)*

(86) Numéro de dépôt international:
**PCT/FR2013/050355**

(87) Numéro de publication internationale:
**WO 2013/124588 (29.08.2013 Gazette 2013/35)**

(54) **MÉTHODE DE CARACTÉRISATION DU COMPORTEMENT MÉCANIQUE DE CIMENTS**

VERFAHREN ZUR CHARAKTERISIERUNG DES MECHANISCHEN VERHALTENS VON ZEMENT

METHOD FOR CHARACTERISATION OF MECHANICAL BEHAVIOUR OF CEMENT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **22.02.2012 FR 1251612**

(43) Date de publication de la demande:
**31.12.2014 Bulletin 2015/01**

(73) Titulaire: **Total SA**
**92400 Courbevoie (FR)**

(72) Inventeurs:
 • **BOIS, Axel-Pierre**
 **69250 Curis-au-Mont-d'Or (FR)**
 • **GARNIER, André**
 **64121 Montardon (FR)**
 • **LAUDET, Jean-Benoît**
 **64018 Pau Cedex (FR)**
 • **VU, Manh-Huyen**
 **69380 Lissieu (FR)**
 • **GHABEZLOO, Siavash**
 **75018 Paris (FR)**
 • **SULEM, Jean**
 **75013 Paris (FR)**

(74) Mandataire: **Cabinet Plasseraud**
**66, rue de la Chaussée d'Antin**
**75440 Paris Cedex 09 (FR)**

(56) Documents cités:
**US-A- 5 412 990    US-B2- 7 089 816**

 • **H LEE: "Ultrasonic in-situ monitoring of setting process of high-performance concrete", CEMENT AND CONCRETE RESEARCH, vol. 34, no. 4, 1 avril 2004 (2004-04-01), pages 631-640, XP055006415, ISSN: 0008-8846, DOI: 10.1016/j.cemconres.2003.10.012**

## Description

### DOMAINE DE L'INVENTION

Le domaine de l'invention est celui de la caractérisation du comportement mécanique des ciments, en particulier des ciments utilisés lors des opérations de cimentation des puits de forage, par exemple dans le cadre de l'exploration et la production d'hydrocarbures. En particulier, l'invention concerne un procédé de détermination de paramètres mécaniques d'un système cimentaire de composition donnée, en fonction du temps, et en fonction de la finesse du système cimentaire, de la pression et/ou de la température.

### ETAT DE LA TECHNIQUE

La cimentation d'un cuvelage dans un puits pétrolier consiste à placer une gaine de ciment dans l'annulaire entre l'extrados du cuvelage et la paroi du trou, le trou pouvant être constitué par un autre cuvelage ou par de la roche. Un bouchon de ciment peut également être créé dans le puits dans le cadre d'opérations de forage ou de complétion, ou au moment du bouchage définitif de celui-ci. Cette gaine ou ce bouchon de ciment ont un rôle primordial dans la stabilité et l'isolation des puits pétroliers.

La gaine ou le bouchon de ciment sont obtenus par pompage d'un laitier ou coulis de ciment fabriqué à partir de ciment, d'eau et d'adjuvants. Ce laitier de ciment est à l'état liquide lorsqu'il est pompé. L'hydratation des particules de ciment conduit le laitier liquide vers un état solide, caractérisé par l'existence d'un squelette et de pores, formant ainsi un milieu poreux.

La gaine de ciment est exposée, durant la vie du puits et sur toute sa longueur, à diverses sollicitations mécaniques et thermiques (conditions in situ) provenant d'opérations menées en surface et dans le puits (tests en pression, changement de boue, stimulations froides et chaudes, production des réserves...) ou de phénomènes prenant naissance directement dans le sous-sol (compaction du réservoir, séismes...), et ce jusqu'à son abandon, voire au-delà. Le bouchon de ciment est également soumis à diverses sollicitations mécaniques et thermiques. Ces sollicitations peuvent endommager le matériau constitutif de la gaine ou du bouchon de ciment, dégrader ses propriétés mécaniques et hydrauliques et, par conséquent, modifier sa contribution à la stabilité et à l'étanchéité du puits.

La connaissance du comportement mécanique du ciment dans les conditions in situ et de l'évolution de ce comportement au cours du temps est essentielle pour l'analyse du fonctionnement du puits pendant son forage, son exploitation, et son bouchage définitif. Par exemple, il est important de connaître le comportement mécanique du ciment pour garantir l'étanchéité du puits lors de ces opérations, lors du stockage et de la séquestration de fluides dans des réservoirs souterrains, tels que des gaz à effet de serre, comme le $CO_2$. Cette connaissance du comportement mécanique du ciment et de son évolution dans le temps, permet notamment de simuler, par le biais de modèles analytico-numériques, la vie des gaines ou des bouchons de ciment. Les conditions in situ influent sur le processus de formation du ciment durci, et par conséquent sur son comportement mécanique. Les ciments obtenus par durcissement de laitiers dans les conditions très spécifiques que sont celles rencontrées dans les puits sont en général très différents des ciments du même type formés dans des conditions ambiantes (c'est-à-dire à l'air ambiant et à pression atmosphérique). Dans les cas extrêmes, pour des puits de 6500 mètres de profondeur, forés avec des boues de densité égale à environ 2, la pression de fond peut atteindre plus de 130 MPa et la température plus de 250°C.

Il existe donc un besoin de connaître les paramètres physiques et mécaniques du ciment et leur évolution dans le temps, à différentes conditions de pression et température, analogues à celles rencontrées le long du puits, et plus particulièrement aux endroits les plus sollicités en pression et/ou en température. Il est à ce titre intéressant d'évaluer ces paramètres après la mise en place de la gaine ou du bouchon de ciment, c'est-à-dire dès le plus jeune âge du ciment lorsque l'hydratation du ciment commence à former une structure, aussi appelée squelette, et ce jusqu'à un temps infini correspondant à un ciment complètement hydraté, dans lequel les processus d'hydratation sont achevés (stabilisation de la composition du ciment, notamment de la quantité d'hydrates).

De nombreuses techniques de mesure sur des échantillons de ciment ont été proposées pour caractériser le comportement mécanique de tels matériaux. Les principaux paramètres mécaniques déterminés à partir de ces mesures sont des paramètres de rupture, tels que la résistance à la compression ou la résistance à la traction (uniaxiale ou triaxiale), et des paramètres de déformabilité, en particulier des paramètres élastiques du matériau tels que le module de Young ou le coefficient de Poisson.

Une première catégorie de techniques recouvre les essais mécaniques statiques (chargement en compression/traction uniaxiale, triaxiale, essai oedométrique etc.) sur des échantillons qui sont durcis à l'intérieur d'un moule dans des bancs de vieillissement, en pression et température, puis qui sont déchargés afin d'être positionnés dans un appareil de mesure. Ces essais nécessitent de ramener les échantillons à pression atmosphérique et à température ambiante, afin de les équiper de capteurs de mesure, de les positionner sous une presse, puis de réaliser les essais proprement dits, après une éventuelle remise des échantillons dans des conditions de température et de pression analogues à celles rencontrées dans le puits. Ces cycles de chargement/déchargement peuvent non seulement endommager les échantillons préalablement à la mesure, mais aussi perturber la mesure des caractéristiques desdits échantillons. En outre, cette première catégorie de techniques ne permet pas de tester les ciments de très jeune âge, car il est nécessaire d'attendre que l'échantillon de ciment puisse être retiré de son moule afin d'être testé. Enfin, de par leur nature destructive, ces techniques ne permettent qu'une mesure ponctuelle dans le temps, et n'offrent pas la possibilité d'accéder à l'évolution des paramètres mécaniques dans le temps, en fonction de l'hydratation du ciment.

Une deuxième catégorie de techniques recouvre les essais dynamiques fondés sur une mesure de la propagation d'ondes ultrasoniques, et ne comprenant pas de retour des échantillons à des conditions ambiantes. Ces techniques ont cependant un intérêt limité de par leur caractère indirect. En particulier, les paramètres statiques, qui sont les paramètres utilisés pour simuler le comportement des gaines ou des bouchons de ciment durant leur vie, ne sont accessibles qu'indirectement : ils sont calculés à partir des paramètres dynamiques, déterminés à partir de la mesure des ondes ultrasoniques, en utilisant des formules de corrélation ; ces formules sont elles-mêmes obtenues à partir d'essais statiques, qui peuvent être entachés d'erreur, voire ne pas couvrir le domaine d'application des matériaux testés. En effet, ces essais statiques sont généralement réalisés sur des échantillons cubiques de ciment dans certaines conditions de pression et de température qui ne correspondent pas nécessairement aux conditions des essais dynamiques. Les mesures de type Ultrasonic Cement Analyser UCA font partie de ces techniques. Une mesure de type UCA consiste à mesurer la vitesse des ondes de compression et à utiliser une corrélation pour évaluer la résistance à la compression du ciment.

Une troisième catégorie de techniques comprend quelques propositions d'essais mécaniques statiques sans étape de chargement/déchargement telle que mentionnée ci-dessus qui nécessitée de ramener l'échantillon de ciment à pression et température ambiante avant d'effectuer une mesure.

Ainsi, le document EP 1541987 décrit un système dans lequel une composition de ciment est coulée dans un moule ayant une forme d'os, où l'échantillon est vieilli sous température et sous pression, et où un chargement en traction uniaxiale est effectué jusqu'à la rupture de l'échantillon, sans avoir à décharger l'échantillon. Toutefois, cette méthode ne permet pas d'effectuer les mesures en conditions in situ, la pression ne pouvant être exercée que sur deux faces de l'échantillon, les autres faces subissant une condition de chargement par réaction du moule et non par application de contraintes en conditions in situ. Les mesures sont donc biaisées. En outre, seuls des tests de traction sont possibles, or ceux-ci sont biaisés en ce qui concerne la mesure des constantes élastiques par rapport aux tests de compression, du fait de l'apparition de microfissures qui invalident l'hypothèse d'élasticité. La plage de détermination des paramètres élastiques est donc très réduite. De plus il n'est pas possible de mesurer les paramètres de rupture en compression, ni de mesurer les paramètres au jeune âge du ciment. Enfin, la géométrie utilisée est non conventionnelle.

Le document US 7,621,186 décrit une variante du système précédent, adoptant une géométrie de type tronconique. Il souffre donc des mêmes inconvénients.

Le document WO 2007/020435 propose une technique qui consiste à faire prendre la composition de ciment dans un annulaire situé entre deux tubes concentriques, puis à faire varier les pressions en intrados du tube intérieur et/ou en extrados du tube extérieur tout en mesurant les déformations induites. Cette technique ne permet pas de réaliser des essais de compression axiale. En outre, cette technique présente l'inconvénient de se fonder sur une mesure en champ de contraintes hétérogène (en élasticité, les champs de contraintes et déformation dans un cylindre creux varient en $1/r^2$). Ainsi la mesure des propriétés élastiques de l'échantillon est très imprécise (très sensible aux erreurs), tout comme celle des propriétés d'endommagement et de rupture de l'échantillon.

Le document US 7,089,816 décrit une technique qui consiste à faire prendre la composition de ciment dans une enveloppe cylindrique, constituée d'une membrane déformable et de deux pistons, et placée dans une enceinte de confinement. Il est ensuite directement procédé aux essais mécaniques en appliquant une pression de confinement au travers de la membrane et un chargement axial par l'intermédiaire des pistons, comme pour une cellule triaxiale classique. Un inconvénient de cette technique est que l'utilisation d'une membrane souple pour la prise du ciment aboutit à un échantillon de forme irrégulière à l'issue de la prise. Du fait des variations de volume associées à la prise, il se produit en effet des instabilités de Taylor conduisant l'échantillon à perdre sa géométrie initiale. Aussi, cette technique ne permet pas d'effecteur des mesures en accord avec les procédures existantes, l'hydratation du ciment n'étant pas correctement reproduite.

Le document US 7,549,320 correspond à une technique du même type, avec une variation de la technologie de chargement. La prise de l'échantillon s'effectue, comme dans US 7,089,816, à l'intérieur d'une membrane flexible. L'enceinte de confinement rigide entourant la membrane flexible est compartimentée grâce à des dispositifs d'étanchéité actifs, et permet l'application d'une pression différentielle à l'échantillon, par l'injection de fluides. La membrane flexible utilisée dans ce dispositif peut en outre être semi-perméable, limitant ainsi la déformation de l'échantillon durant sa prise, mais perturbant le processus d'hydratation de l'échantillon de ciment.

Le document US 7,552,648 décrit encore une autre variante, dans laquelle on injecte un fluide dans l'échantillon lui-même, qui est poreux, afin d'obtenir la pression souhaitée. On effectue ensuite un essai de traction. Aucun test de compression n'est prévu, et l'apport de fluide extérieur ne simule pas correctement les échanges hydriques en conditions in situ, comme c'est aussi le cas pour la technique selon le document US 7,549,320.

Outre les inconvénients mentionnés ci-dessus pour cette troisième catégorie de techniques, ce type d'essais ne permet pas d'effectuer des mesures sur des ciments de très jeune âge, c'est-à-dire dès les premières heures de l'hydratation, ni de suivre l'évolution des paramètres mécaniques mesurés dans le temps, en fonction de l'hydratation du ciment.

Aucune des trois catégories de techniques décrites ne permet de mesurer sans artéfacts les propriétés mécaniques d'un ciment en cours de prise.

## OBJECTIFS DE L'INVENTION

Il existe par conséquent un besoin de disposer d'un procédé de détermination des paramètres mécaniques d'un système cimentaire et de leur évolution dans le temps, dans des conditions in situ, qui ne comporte pas les inconvénients des techniques de mesure décrites ci-dessus.

L'invention vise à répondre à un besoin de caractérisation simple du comportement mécanique d'un système cimentaire, à travers la détermination de propriétés mécaniques statiques et de propriétés hydro-mécaniques exprimées en fonction du temps, et en fonction de différentes pressions et températures correspondant aux conditions rencontrées le long d'un puits de forage.

Un autre objectif de l'invention consiste à fournir un procédé facilement industrialisable pour caractériser le comportement mécanique d'un système cimentaire, qui utilise des mesures simples et normalisables.

Un autre objectif de l'invention consiste à fournir un procédé permettant d'estimer l'évolution des paramètres mécaniques d'un système cimentaire au cours du temps, du début à la fin de son hydratation.

L'invention vise également à fournir une estimation de paramètres mécaniques statiques du ciment, qui constituent les paramètres utilisés pour simuler de manière fiable le comportement mécanique des gaines ou des bouchons de ciment durant leur vie.

## BREVE DESCRIPTION DES DESSINS

La figure 1 représente le logigramme du procédé selon l'invention.

La figure 2 est un schéma illustrant les différents stades du processus d'hydratation d'un ciment Portland.

La figure 3 présente un exemple de dispositif de type Ultrasonic Cement Analyser (UCA) permettant de mesurer la vitesse des ondes de compression dans un échantillon de ciment utilisée dans le procédé selon l'invention.

La figure 4 est un diagramme représentant la vitesse des ondes de compression en fonction du temps $V_p(t)$ mesurée lors d'un essai de type UCA sur un système cimentaire comprenant du ciment Portland, ainsi que le degré d'hydratation en fonction du temps $\alpha(t)$ du même système cimentaire mesuré lors d'un essai calorimétrique.

La figure 5 est un diagramme illustrant la relation linéaire entre la vitesse des ondes de compression en fonction du temps $V_p(t)$ et le degré d'hydratation en fonction du temps $\alpha(t)$ tels que représentés à figure 3.

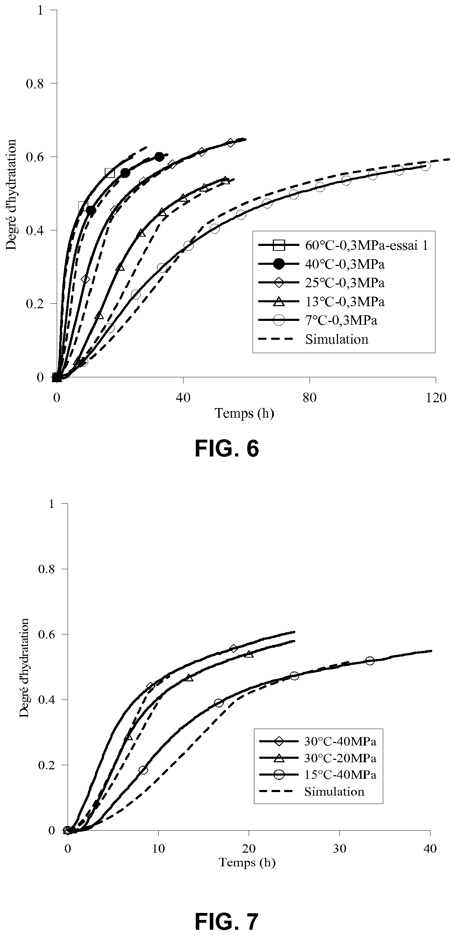

Les figures 6 à 8 sont des diagrammes comparant le degré d'hydratation en fonction du temps $\alpha(t)$ déterminé par le procédé selon l'invention pour un système cimentaire donné, et le degré d'hydratation en fonction du temps $\alpha(t)$ pour ce même système, déterminé expérimentalement. Les degrés d'hydratation sont représentés pour différentes valeurs de pression et de température. Dans la figure 6, $\alpha(t)$ est représenté pour une pression faible de 0.3 MPa et à différentes températures (7°C, 13°C, 25°C, 40°C et 60°C). Dans la figure 7, $\alpha(t)$ est représenté à des pressions plus élevées, selon les couples pression/température suivants : (40MPa, 30°C), (40MPa, 15°C), (20MPa, 30°C).

Sur la figure 8, $\alpha(t)$ est représenté pour les deux couples pression/température suivants : (0.3MPa, 60°C), (20MPa, 60°C),

La figure 9 est un diagramme illustrant la composition d'un système cimentaire donné en fonction du degré d'hydratation du système, déterminé par le procédé selon l'invention. En particulier, la fraction volumique des différents constituants du système cimentaire en fonction du degré d'hydratation du système cimentaire est donnée, pour un couple de pression et de température fixé.

Les figures 10 à 13 sont des diagrammes comparant les paramètres mécaniques en fonction du temps déterminés par le procédé selon l'invention, pour un système cimentaire donné, à ceux déterminés expérimentalement et issus de la littérature (Boumiz et al.[1], Haecher et al.[2]) pour ce même système cimentaire.

## DESCRIPTION DETAILLEE DE L'INVENTION

Dans la description de l'invention et les exemples particuliers de l'invention qui suivent, il est fait référence aux dessins annexés.

Pour atteindre au moins l'un des objectifs susvisés, parmi d'autres, la présente invention propose un procédé de détermination de paramètres mécaniques d'un système cimentaire de composition initiale $C_0$ et de finesse $\Phi$, en fonction du temps, et en fonction de la finesse du système cimentaire, de la pression et/ou de la température. Ce procédé comprend les étapes suivantes :

(A) déterminer le degré d'hydratation du système cimentaire en fonction du temps $\alpha(t)$ à partir de la vitesse des ondes de compression en fonction du temps $V_p(t)$ mesurée dans un échantillon du système cimentaire, à une pression $P_1$ et une température $T_1$ ;

(B) déterminer le degré d'hydratation $\alpha(t)$ en fonction de valeurs souhaitées de finesse $\Phi_n$ du système cimentaire, de pression $P_n$ et/ou de température $T_n$ ;

(C) déterminer la composition du système cimentaire en fonction du temps $C(t)$ et en fonction de valeurs souhaitées de finesse $\Phi_n$ du système cimentaire, de pression $P_n$ et/ou de température $T_n$, à partir du degré d'hydratation $\alpha(t)$ déterminé à l'étape B ;

(D) déterminer au moins un paramètre mécanique du système cimentaire en fonction du temps, et en fonction de valeurs souhaitées de finesse $\Phi_n$ du système cimentaire, de pression $P_n$ et/ou de température $T_n$, à partir de la composition du système cimentaire $C(t)$ déterminée à l'étape C.

Selon un mode de réalisation de l'invention, le procédé permet de caractériser le comportement mécanique d'un système cimentaire utilisé en tant que gaine ou bouchon de ciment dans un puits, de préférence un puits pétrolier. Le procédé selon l'invention ne se limite pas à cette utilisation, et peut être mis en oeuvre dans le cadre de toute construction de puits comprenant une gaine ou un bouchon de ciment pour lequel/laquelle la caractérisation du comportement mécanique du système cimentaire utilisé est souhaitée, par exemple dans le domaine du stockage de gaz ou de la géothermie.

*Définitions*

Dans tout le présent exposé, sauf indication expresse contraire, un singulier doit être interprété comme un pluriel et réciproquement.

Les définitions qui suivent sont données à titre d'exemples, pour faciliter l'interprétation du présent exposé.

Un *système cimentaire* au sens de la présente invention correspond à un laitier de ciment ou un ciment durci.

Par *laitier de ciment*, on entend une composition fluide (liquide, pâteuse, granulaire...) à base de ciment et d'eau, apte à durcir, c'est-à-dire susceptible de passer à un état solide ou quasiment solide au cours du temps (en subissant une étape de durcissement). L'expression « coulis de ciment » est utilisée en tant que synonyme de laitier de ciment. Ainsi, le durcissement correspond dans ce cas essentiellement à l'hydratation (ou prise) de la composition à base de ciment et d'eau.

Par *ciment durci*, on entend un laitier de ciment qui a subit un durcissement, et dans lequel a commencé à se développer un squelette poreux, pouvant lui conférer une résistance mécanique. Au sens de l'invention, un ciment durci n'est pas nécessairement un ciment complètement durci, c'est-à-dire dont l'hydratation est achevée. Un ciment complètement durci est un ciment dont l'hydratation est complète à 100%.

Par *paramètre mécanique statique*, on entend un paramètre qui n'est pas obtenu à partir de la mesure de vitesses d'ondes. Les paramètres statiques constituent les paramètres utilisés pour simuler de manière fiable le comportement mécanique des gaines ou des bouchons de ciment durant leur vie, à l'inverse des paramètres dynamiques. Ces derniers, généralement issus de méthodes de mesures d'ondes soniques/ultrasoniques, divergent des paramètres statiques, en raison de la dépendance des propriétés mécaniques du ciment à la fréquence des ondes utilisées et de la dimension des échantillons de ciment testés.

Par *finesse d'un ciment*, on entend la finesse de broyage d'un ciment exprimée par sa surface spécifique (surface développée par unité de masse, exprimée en $cm^2/g$), et mesurée à l'aide d'un appareil de type Blaine selon la norme NF EN 196-6.

Par *ondes de compression*, ou *ondes P*, on entend des ondes soniques ou ultrasoniques crées par la vibration mécanique d'un support fluide ou solide et propagée grâce à l'élasticité du milieu environnant sous forme d'ondes longitudinales.

Par les termes *initial(aux), initiale(s),* utilisés par exemple en référence à la composition du système cimentaire, à la température, la pression ou tout autre paramètre physico-chimique dont il est question dans la présente description, il est fait référence au stade initial du système cimentaire sous forme d'un laitier de ciment, et dans lequel les réactions d'hydratation n'ont pas ou quasiment pas débutées, correspondant au temps initial t=0.

Par *phase initiale réactive,* on entend une phase initiale du ciment du système cimentaire, apte à s'hydrater pour donner une phase hydratée, aussi appelée hydrate dans la présente description.

*Le système cimentaire*

Le procédé selon la présente invention s'applique à l'ensemble des ciments dont l'hydratation se fait au travers d'une série de réactions chimiques exothermiques entre ses composants et l'eau, par exemple les ciments Portland, les ciments alumineux, et autres ciments définis selon les normes EN-196-2, EN-196-6, EN196-7, ISO 3310-1, ISO 13500, et les normes ISO 10426-1 à ISO 10426-6 en ce qui concerne l'industrie pétrolière.

Le système cimentaire selon la présente invention peut comprendre des adjuvants, permettant d'obtenir les propriétés désirées :

- des accélérateurs (exemple : le chlorure de calcium), qui ont pour objectifs de réduire la période d'induction du système cimentaire, et éventuellement d'accélérer le développement de la résistance à la compression uniaxiale ;
- des retardateurs (exemple : les lignosulfonates), qui ont pour objectif d'augmenter la période d'induction du système cimentaire ;
- des extendeurs (exemple : la bentonite, des billes creuses), qui ont pour objectif de diminuer la densité du laitier de ciment et/ou réduire la concentration en ciment ou augmenter la concentration d'eau ;
- des alourdisseurs (exemple : l'hématite) qui ont pour objectif d'augmenter la densité du laitier de ciment ;
- des dispersants (exemple : les lignosulfonates), qui ont pour objectif de diminuer la viscosité du laitier de ciment en défloculant les grains de ciment ;
- des contrôleurs de filtrat (exemple : le latex), qui ont pour objectif de limiter les transferts d'eau du laitier de ciment vers le système extérieur par construction d'un cake peu ou pas perméable contre la formation ;
- des contrôleurs de pertes (exemple : la gilsonite), qui ont pour objectif de limiter les pertes du ciment dans les zones très perméables ;
- du sel ;
- des additifs spéciaux, comme des anti-mousses, des fibres, des agents d'expansion, des agents anti-gaz, des agents anti-sédimentation, ou des agents donnant des ciments résilients.

Le système cimentaire selon la présente invention comprend une composition initiale $C_0$ à l'instant t = 0. Cette composition initiale $C_0$ comprend les phases initiales du ciment, parmi lesquelles au moins une phase initiale réactive pouvant s'hydrater, et l'eau initiale, selon des fractions données, par exemples exprimées en volume. A l'instant t, l'hydratation du système cimentaire est avancée, et la composition du ciment C(t) est différente de la composition initiale $C_0$. La composition du ciment C(t) comprend m phases, parmi lesquelles les phases initiales du ciment dans des proportions différentes de $C_0$, au moins une phase hydratée issue de l'hydratation d'au moins une phase initiale réactive du ciment, et de l'eau.

Selon un mode de réalisation de l'invention, le ciment du système cimentaire comprend au moins une phase initiale réactive X, de préférence choisie dans le groupe constitué par le silicate tricalcique de formule $3CaO.SiO_2$ (Alite), de symbole $C_3S$, le silicate bicalcique de formule $2CaO.SiO_2$ (Belite), de symbole $C_2S$, l'aluminate tricalcique de formule $3CaO.Al_2O_3$ (Aluminate), de symbole $C_3A$, l'aluminoferrite tétracalcique $C_4F$, et leurs associations. Selon ce mode de réalisation, le ciment du système cimentaire comprend au moins une phase hydratée issue de l'hydratation d'au moins une phase initiale réactive du ciment, de préférence choisie dans le groupe constitué par le silicate de calcium hydraté C-S-H, l'hydroxyde de calcium CH, le trisulfoaluminate de calcium hydraté TSA (Ettringite), le monosulfate de calcium hydraté AFm, l'aluminoferrite de calcium hydraté, et leurs associations.

Les phases initiales telles que les silicates $C_3S$, $C_2S$ et les aluminates sont des phases réactives qui réagissent avec l'eau du système cimentaire pour donner des phases hydratées, par le biais de réactions chimiques exothermiques. Les silicates réagissent avec l'eau pour former des silicates de calcium hydratés C-S-H, et de l'hydroxyde de calcium CH (Portlandite de formule $Ca(OH)_2$). Les aluminates réagissent avec l'eau et le gypse, ou avec l'eau seule pour former les aluminates hydratés suivants : le trisulfoaluminate de calcium hydraté TSA (Ettringite), le monosulfate de calcium hydraté AFm et l'aluminoferrite de calcium hydraté.

La microstructure du système cimentaire, en particulier du ciment durci, est très complexe. Les fractions volumiques, la possibilité de cristallisation, la morphologie et les dimensions des phases hydratées du ciment durci sont présentées dans le tableau 1 ci-dessous, relatif à un ciment Portland (Bernard et al.[12]).

**TABLEAU 1**

| Phase minéralogique | Fraction volumique (%) | Possibilité de cristallisation | Morphologies | Dimensions |
|---|---|---|---|---|
| C-S-H | 50-70 | Très peu | Non résolu | 10-100 $\mu$m |
| CH | 15-20 | Très bien | Solide hexagonal | 10-100 $\mu$m |

(suite)

| Phase minéralogique | Fraction volumique (%) | Possibilité de cristallisation | Morphologies | Dimensions |
|---|---|---|---|---|
| Ettringite | 1-5 | Bien | Solide de forme des aiguilles | 10x0,5 $\mu$m |
| Monosulfoaluminate | 1-5 | bien | Solide sous forme des plans hexagonaux | 1x1x0,1 $\mu$m |

La Porlandite CH est formée de cristaux hexagonaux larges qui sont entourés par la phase C-S-H, qui est sous forme de gels, et occupe environ 20% du volume du ciment durci. Les monosulfoaluminates et l'Ettringite jouent un rôle mineur dans la structure du ciment durci et représentent généralement une fraction volumique de 15% à 20%. La phase C-S-H constitue le produit majeur du ciment durci. La phase C-S-H est une phase poreuse, de structure amorphe et colloïdale, et présente une composition chimique variée. Elle occupe entre 50% et 70% du volume total du ciment durci, ce qui donne aux propriétés de cette phase un rôle dominant dans les propriétés macroscopiques du système cimentaire. La phase C-S-H existe sous deux formes : la phase "haute densité" C-S-H HD (aussi dénommée « HD » dans la présente description) et la phase "faible densité" C-S-H LD (aussi dénommée « LD » dans la présente description). Chacune de ces deux formes possède, selon Jennings et al.[8,9], une unité commune appelée globule. La différence entre ces deux types de C-S-H consiste en l'arrangement des globules, résultant en différentes porosités selon le type de C-S-H : environ 24% pour le C-S-H HD, avec une taille de pores comprise entre environ 10nm et 100nm (Constantinidcs[10]), et environ 37% pour le C-S-H LD, avec une taille de pores comprise entre environ 5nm et 50nm.

Selon un mode de réalisation de l'invention, le système cimentaire comprend du ciment Portland, qui constitue l'un des ciments les plus courants, comprenant les phases initiales suivantes utilisés (voir les normes EN-196-2, EN-196-6, EN196-7, ISO 3310-1, ISO 13500, et les normes ISO 10426-1 à ISO 10426-6 pour les teneurs en phases initiales, dépendant de la classe et du grade du ciment) :

- du silicate tricalcique de formule $3CaO.SiO_2$ (Alite), de symbole $C_3S$ ;
- du silicate bicalcique de formule $2CaO.SiO_2$ (Belite), de symbole $C_2S$ ;
- de l'aluminate tricalcique de formule $3CaO.Al_2O_3$ (Aluminate), de symbole $C_3A$ ;
- du ferro-aluminate tétracalcique de formule $4CaO.Al_2O_3\,Fe_2O_3$ (Ferrite), de symbole $C_4F$ ;
- du sulfate dihydraté de calcium $CaSO_4.2H_2O$ (gypse).

Les phases silicatées et les aluminates sont issus d'un clinker broyé, principal constituant du ciment Portland.

Il est bien entendu que le procédé selon l'invention ne se limite pas au système cimentaire comprenant un ciment Portland. En particulier, le procédé peut être adapté pour la détermination de paramètres mécaniques de systèmes cimentaires variés, présentant des compositions chimiques et des réactions d'hydratation différentes de celles d'un système cimentaire comprenant un ciment Portland, sans pour autant sortir du cadre de l'invention.

*Le processus d'hydratation du système cimentaire*

Le procédé selon l'invention prend en compte différentes phases du processus d'hydratation d'un système cimentaire pour la détermination d'au moins un paramètre mécanique de celui-ci. Le processus d'hydratation peut être suivi par l'évolution du flux de chaleur crée par les réactions exothermiques d'hydratation des phases initiales réactives du ciment avec l'eau.

La quantité de chaleur dégagée par les réactions exothermiques lors de l'hydratation du système cimentaire peut être mesurée en utilisant un calorimètre. Deux types de calorimètre sont généralement utilisés. Le premier type correspond aux calorimètres quasi adiabatiques, dans lesquels la réaction a lieu à l'intérieur d'une enceinte parfaitement isolée thermiquement. Ce type de calorimètre n'est pas adapté à la mesure d'échantillons de systèmes cimentaires sous pressions élevées, et la pression limite d'utilisation est d'environ 0,1MPa. Un deuxième type correspond aux calorimètres isothermes, avec lesquels les essais sont réalisés dans un espace de l'ordre du centimètre cube, et où la température est contrôlée afin que la température de l'espace ne varie pas. Les matériaux utilisés pour ce type d'appareil de mesure permettent de travailler avec des pressions plus élevées, de l'ordre du Méga Pascal.

En général, plusieurs stades peuvent être identifiés au cours du processus d'hydratation d'un système cimentaire. Classiquement, l'hydratation du système cimentaire comprend dans cet ordre :

- une période de dissolution, au cours de laquelle se produit la dissolution de l'Ettringite et la formation de gel CSH ;
- une période dormante, dite aussi période d'induction, au cours de laquelle se produit une augmentation de la

concentration en $C_3A$ et en ions $OH^-$;

- une période de formation rapide de C-S-H et de Portlandite CH, dite aussi période d'accélération ;
- une période de ralentissement de la formation de C-S-H et de Portlandite CH ; et de formation éventuelle de monosulfoaluminate ;
- et une période de cure, dite aussi période de diffusion, où les réactions chimiques sont contrôlées par des phénomènes de diffusion.

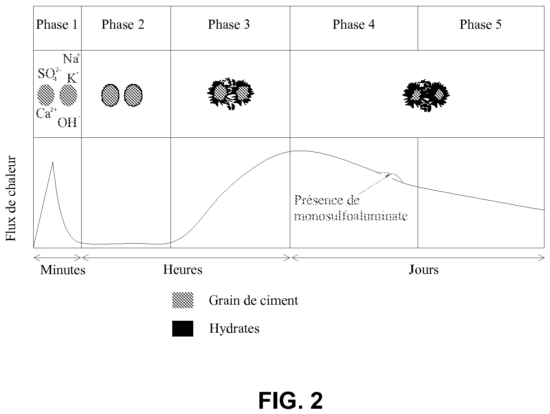

La figure 2 illustre le processus d'hydratation dans le cas d'un ciment Portland (Rixom et al.[15]). Dans ce cas, le processus d'hydratation est généralement divisé en 5 étapes. Chaque étape correspond à une tendance d'évolution de la chaleur provoquée par les réactions d'hydratation :

- Période 1 : une période d'hydratation initiale se déroule pendant environ 15 minutes, durant laquelle les composants chimiques les plus solubles ($Na_2SO_4$, $K_2SO_4$, $CaSO_4.xH_2O$) du ciment se dissolvent, faisant augmenter le pH du laitier de ciment.
- Période 2 : une période dormante s'ensuit, pendant environ 4 heures. La chaleur d'hydratation n'évolue presque pas. La résistance de la pâte de ciment n'est pas encore créée.
- Période 3 : cette troisième période correspond à une accélération de l'hydratation, qui dure environ 4 heures. Au cours de celle-ci, la chaleur d'hydratation évolue très rapidement. Les hydrates formés se mettent en contact, ce qui forme la résistance de la pâte.
- Période 4 : une période de ralentissement s'opère ensuite et dure quelques jours. L'évolution de la chaleur d'hydratation ralentit.
- Période 5 : une période de cure se manifeste pendant plusieurs mois après le début de la prise du système cimentaire, et l'évolution de la quantité des hydrates se stabilise progressivement.

L'évolution de l'hydratation du système cimentaire dans le temps est classiquement décrite par l'évolution du degré d'hydratation dans le temps $\alpha(t)$, qui est un paramètre intrinsèque, et dont la valeur varie de 0 à 1. Celui-ci, à un instant t, peut être évalué comme le rapport de la quantité de chaleur $Q(t)$ produite par les réactions chimiques à l'instant t sur la quantité totale de chaleur $Q(t=\infty)$ produite lorsque les réactions chimiques ont toutes eu lieu. La cinétique d'hydratation varie au cours du processus d'hydratation. Parmi les périodes du processus d'hydratation, un premier stade où l'hydratation est gouvernée principalement par un processus de nucléation et de croissance, tel que décrit par exemple par Avrami[3], est classiquement reconnu. Un deuxième stade du processus d'hydratation du système cimentaire est caractérisé par une hydratation gouvernée principalement par un processus diffusion des ions. Ce deuxième stade commence lorsque le degré d'hydratation $\alpha$ atteint une valeur d'un degré d'hydratation seuil $\alpha^*$. Lors du premier stade, gouverné par les phénomènes de nucléation et de croissance, une couche d'hydrates se crée autour des grains de ciment, et est relativement perméable, ce qui permet la diffusion des ions et l'avancement des réactions chimiques. Lorsqu'un degré d'hydratation seuil $\alpha^*$ est atteint, une diminution de la diffusion des ions se produit, liée à l'épaississement de la couche d'hydrates autour du grain de ciment et à la baisse de perméabilité de cette couche. C'est alors la diffusion des ions qui contrôle l'avancement des réactions (Kondo et Kodama[4] ; Fuji et Kondo[5]). Dans le cas du processus d'hydratation d'un ciment Portland, tel que représenté à la figure 2, le degré d'hydratation seuil $\alpha^*$ marque le passage de la période 4 à la période 5. Les périodes 3 et 4 correspondent aux phases de nucléation et de croissance selon Avrami[3], et la période 5 (période de cure) est principalement gouvernée par le processus de diffusion des ions.

Le silicate tricalcique $C_3S$ et le silicate bicalcique $C_2S$ réagissent avec l'eau selon les équations suivantes, issues de Jennings et al.[6]:

$$2C_3S + 10{,}6H \rightarrow C_{3,4} - S - H_8 + 2{,}6CH \text{ (I)} \quad 2C_2S + 8{,}6H \rightarrow C_{3,4} - S_2 - H_8 + 0{,}6CH$$

Les phases hydratées C-S-H et le CH forment les principaux constituants du ciment durci, et sont principalement responsables de la solidité du ciment durci. La cinétique d'hydratation de la phase $C_3S$ est plus rapide que celle de la phase $C_2S$, et la quantité de CH créée par l'hydratation du $C_3S$ est environ trois fois plus grande que celle crée par l'hydratation de $C_2S$. Ainsi, la phase $C_3S$ joue un rôle majeur dans le processus d'hydratation et dans le développement du comportement mécanique du système cimentaire, en initiant la résistance de la pâte de ciment pendant les premiers jours.

Selon un mode de réalisation de l'invention, le procédé prend en compte les deux réactions *(I)* décrites ci-dessus pour simuler le processus d'hydratation.

*Nature des paramètres mécaniques déterminés par le procédé selon l'invention*

L'invention vise à caractériser le comportement mécanique d'un système cimentaire, et notamment à déterminer certains paramètres mécaniques et leur évolution dans le temps, dans des conditions in situ, qui pourront ensuite être utilisés, par exemple, dans la modélisation du comportement des gaines ou des bouchons de ciment des puits de forage durant leur vie. Le procédé selon l'invention permet de déterminer au moins un paramètre mécanique du système cimentaire en fonction du temps, pour des valeurs souhaitées de finesse $\Phi_n$ du système cimentaire, de pression $P_n$ et/ou de température $T_n$.

Selon un mode de réalisation de l'invention, le procédé permet de déterminer au moins un paramètre mécanique qui est choisi parmi les paramètres de déformabilité statiques, de préférence les paramètres élastiques statiques tel que module de Young statique E, le coefficient de Poisson statique v, le module d'incompressibilité K, le module de cisaillement G, et leurs combinaisons, comme par exemple le module oedométrique $K_v$, ou le module de Lamé $\lambda$. De manière préférée, deux paramètres élastiques statiques sont déterminés. Avantageusement, il est possible de décrire les propriétés élastiques du système cimentaire, déterminées de manière unique par la combinaison de deux paramètres élastiques statiques, en utilisant des formules bien connues en élasticité, telles que décrites dans le tableau 2 ci-dessous.

**TABLEAU 2**

| K | E | $\lambda$ | $\nu$ | K$_v$ | G |
|---|---|---|---|---|---|
| $\lambda + \dfrac{2G}{3}$ | $G\dfrac{3\lambda+2G}{\lambda+G}$ | - | $\dfrac{\lambda}{2(\lambda+G)}$ | $\lambda + 2G$ | - |
| - | $9K\dfrac{K-\lambda}{3K-\lambda}$ | - | $\dfrac{\lambda}{3K-\lambda}$ | $3K - 2\lambda$ | $\dfrac{3}{2}(K-\lambda)$ |
| - | $\dfrac{9KG}{3K+G}$ | $K-\dfrac{2G}{3}$ | $\dfrac{3K-2G}{2(3K+G)}$ | $K+\dfrac{4G}{3}$ | - |
| $\dfrac{EG}{3(3G-E)}$ | - | $G\dfrac{E-2G}{3G-E}$ | $\dfrac{E}{2G}-1$ | $G\dfrac{4G-E}{3G-E}$ | - |
| - | - | $3K\dfrac{3K-E}{9K-E}$ | $\dfrac{3K-E}{6K}$ | $3K\dfrac{3K+E}{9K-E}$ | $\dfrac{3KE}{9K-E}$ |
| $\lambda\dfrac{1+\nu}{3\nu}$ | $\lambda\dfrac{(1+\nu)(1-2\nu)}{\nu}$ | - | - | $\lambda\dfrac{1-\nu}{\nu}$ | $\lambda\dfrac{1-2\nu}{2\nu}$ |
| $2G\dfrac{1+\nu}{3(1-2\nu)}$ | $2G(1+\nu)$ | $2G\dfrac{\nu}{1-2\nu}$ | - | $2G\dfrac{1-\nu}{1-2\nu}$ | - |
| - | $3K(1-2\nu)$ | $3K\dfrac{\nu}{1+\nu}$ | - | $3K\dfrac{1-\nu}{1+\nu}$ | $3K\dfrac{1-2\nu}{2(1+\nu)}$ |
| $\dfrac{E}{3(1-2\nu)}$ | - | $\dfrac{E\nu}{(1+\nu)(1-2\nu)}$ | - | $\dfrac{E(1-\nu)}{(1+\nu)(1-2\nu)}$ | $\dfrac{E}{2(1+\nu)}$ |

Selon un mode de réalisation de l'invention, au moins un paramètre mécanique déterminé selon l'étape D, en particulier choisi parmi les paramètres de déformabilité statiques, de préférence les paramètres élastiques statiques, est un paramètre en conditions drainées. Dans un système drainé, il est fait l'hypothèse d'une circulation des fluides de pores, par exemple des liquides, entre le système cimentaire et son environnement (encaissant) afin de conserver la pression de pores constante. A l'opposé, dans un système non drainé, il est fait l'hypothèse d'aucun échange de fluides de pores entre le système cimentaire et son environnement. La valeur des paramètres mécaniques du système cimentaire, en particulier des paramètres de déformabilité statique, sera différente selon que le système est drainé ou non drainé.

Selon un autre mode de réalisation, le procédé comprend en outre la détermination d'au moins un paramètre de couplage hydro-mécanique, tel que le coefficient de Biot b ou le coefficient de Skempton B, et leurs combinaisons. La connaissance de tels paramètres de couplage hydro-mécanique permet notamment de déterminer les paramètres de déformabilité statiques, de préférence les paramètres élastiques statiques, en conditions non drainées à partir de la connaissance des paramètres élastiques statiques, en conditions drainées. Ces paramètres peuvent, par exemple, être utilisés pour simuler le comportement d'une gaine de ciment dont les pores sont saturés en eau lorsque la vitesse de chargement est nettement plus rapide que la vitesse de diffusion des pressions de pores. L'ensemble des formules *(II)* utilisées pour passer des propriétés élastiques statiques drainées (indice d) à celles statiques non-drainées (indice u), et réciproquement, est donné ci-dessous.

$$v_d = \frac{3v_u - (1+v_u)\cdot bB}{3 - 2(1+v_u)\cdot bB}$$

$$v_u = \frac{3v_d - (1+2v_d)\cdot bB}{3 - (1-2v_d)\cdot bB}$$

$$E_d = \frac{3(1-bB)}{3 - 2(1+v_u)\cdot bB}\cdot E_u \qquad\qquad (II)$$

$$E_u = \frac{3E_d}{3 - (1-2v_d)\cdot bB}$$

$$K_d = (1-bB)\cdot K_u$$

$$K_u = \frac{K_d}{1-bB}$$

Le module de Young E et le coefficient de Poisson $v$ sont des constantes élastiques qui sont classiquement déterminées lors d'un essai de compression uniaxiale ou triaxiale où un échantillon cylindrique d'élancement 2 est soumis à une augmentation de la contrainte axiale alors qu'aucun confinement n'est appliqué et que la pression de pores est constante. Ces coefficients sont alors définis classiquement, dans la zone où les déformations sont réversibles, par les formules *suivantes :* $E = \Delta\sigma_{axial} / \Delta\varepsilon_{axial}$, $v = -\Delta\varepsilon_{radial} / \Delta\varepsilon_{axial}$, avec $\Delta\sigma_{axial}$ l'incrément de contrainte axiale, $\Delta\varepsilon_{axial}$ l'incrément de déformation axiale et $\Delta\varepsilon_{radial}$ l'incrément de déformation radiale.

Le module d'incompressibilité K est une constante élastique classiquement déterminée lors d'un essai de compression isotrope où un échantillon est soumis à une augmentation de contrainte axiale égale à l'augmentation de confinement (dite contrainte isotrope) alors que la pression de pores est constante. Ce coefficient est alors défini classiquement, dans la zone où les déformations sont réversibles, par la formule suivante : $K = \Delta\sigma_{isotrope} / \Delta\varepsilon_{volumique}$, avec $\Delta\sigma_{isotrope}$ l'incrément de contrainte isotrope et $\Delta\varepsilon_{volumique}$ l'incrément de déformation volumique.

Le module de cisaillement G n'est généralement pas mesuré directement, bien qu'il puisse l'être théoriquement, et est évalué à partir de la connaissance de deux autres paramètres élastiques.

Le module oedométrique, $K_v$ est une constante élastique classiquement déterminée lors d'un essai de compression oedométrique où un échantillon cylindrique est soumis à une augmentation de la contrainte axiale alors qu'aucun déplacement radial n'est autorisé et que la pression de pores est constante. Ce coefficient est alors défini classiquement, dans la zone où les déformations sont réversibles, par la formule suivante : $K_v = \Delta\sigma_{axial} / \Delta\varepsilon_{axial}$, avec $\Delta\sigma_{axial}$ l'incrément de contrainte axiale et $\Delta\varepsilon_{axial}$ l'incrément de déformation axiale.

Le procédé selon l'invention permet de déterminer les paramètres de déformabilité statiques, qui ont pour avantage d'être ceux utilisés dans la modélisation des gaines ou des bouchons de ciment des puits de forage. Ces paramètres statiques ne doivent pas être confondus avec ceux dits « dynamiques », qui sont évalués à partir de la connaissance de la vitesse des ondes de compression et de cisaillement.

Les paramètres de couplage hydro-mécanique sont par exemple le coefficient de Biot (b) et le coefficient de Skempton (B). Le coefficient de Biot (b) est le coefficient qui permet de définir la contrainte effective, c'est-à-dire la contrainte qui induit la déformation d'un matériau dans le cas où la pression de pores n'est pas nulle, et s'exprime dans la relation suivante : $\Delta\sigma' = \Delta\sigma - b \cdot P_p$, où σ' est la contrainte effective, σ est la contrainte totale, et $P_p$ est la pression de pores. Le coefficient de Skempton (B) est le coefficient qui permet de calculer la variation de pression due à une variation de contrainte isotrope sans expulsion de fluide pour un échantillon saturé, et s'exprime selon la relation $\Delta P_p = B \cdot \Delta\sigma$, dans laquelle $\Delta\sigma$ est la variation de contrainte isotrope , et $\Delta P_p$ est la variation de pression de pores.

Selon le procédé de l'invention, ces paramètres peuvent être déterminés pour le système cimentaire dès son plus jeune âge, ce qui permet notamment une meilleure modélisation du comportement mécanique du système cimentaire. Il peut être important de connaître le comportement du ciment au stade précoce dans le cadre des opérations de forage, par exemple pour des opérations qui doivent pouvoir être effectuées rapidement après le pompage du laitier de ciment dans le puits, telles que des logs thermiques permettant de localiser le sommet de la colonne de ciment, des opérations de forage supplémentaires une fois le tubage posé, la perforation des zones de productions, ou encore pour évaluer l'état de contraintes dans la gaine de ciment une fois le ciment durci.

*Le procédé*

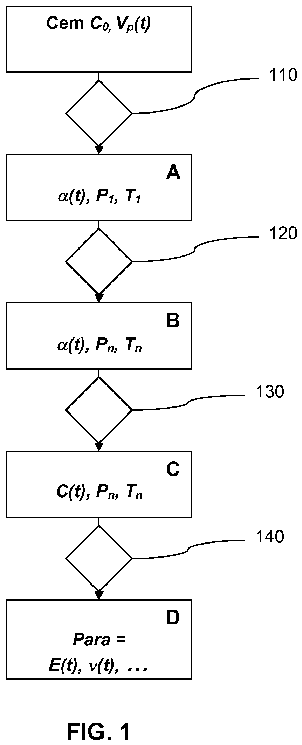

L'organigramme de la figure 1 représente schématiquement le procédé selon l'invention, qui comprend les étapes A à D décrites ci-dessous.

Le système cimentaire testé par le procédé selon l'invention présente une composition initiale $C_0$ connue et une finesse Φ. Un autre paramètre initial utilisé par le procédé selon l'invention est la vitesse des ondes de compression dans le système cimentaire en fonction du temps Vp(t). La connaissance de ces paramètres initiaux permet, grâce au procédé selon l'invention, de déterminer au moins un paramètre mécanique du système cimentaire.

Le procédé selon l'invention ne se limite pas à la combinaison des étapes A à D, selon l'ordre cité, et des étapes intermédiaires peuvent être réalisées, sans pour autant sortir du cadre de l'invention.

*Etape de mesure de la vitesse des ondes de compression en fonction du temps du système cimentaire*

Selon un mode de réalisation de l'invention, le procédé comprend en outre une étape initiale de mesure de la vitesse des ondes de compression en fonction du temps Vp(t) dans un échantillon du système cimentaire. Une telle mesure peut, par exemple, être réalisée selon une méthode de type Ultrasonic Cement Analyser UCA, telle que par exemple décrite dans le brevet US 4,259,868. Les méthodes de type UCA sont des méthodes bien connues pour évaluer la qualité d'un système cimentaire, et font partie de la deuxième catégorie de méthodes de mesure décrite plus haut, permettant de caractériser le comportement mécanique d'un système cimentaire. Il s'agit de méthodes non destructives permettant d'analyser des échantillons de ciment, sous forme coulis de ciment, en utilisant la mesure de la vitesse d'ondes ultrasoniques, notamment de compression, traversant l'échantillon, sans nécessiter de ramener les échantillons dans les conditions environnementales (pression et température) du laboratoire préalablement à la mesure. Une amélioration des méthodes de type UCA permet en outre de mesurer les vitesses des ondes de cisaillement, et sont désignées en tant que mesures MPRO (Reddy et al.[7]). La vitesse des ondes de compression et de cisaillement en fonction du temps ainsi enregistrée permet de calculer d'une part la résistance à la compression uniaxiale, reliée à la vitesse des ondes de compression par une fonction de corrélation, et d'autre part les paramètres élastiques dynamiques, à partir des relations suivantes :

Coefficient de poisson dynamique :

$$v_{dyn} = \frac{\dfrac{1}{2} - \left(\dfrac{V_S}{V_P}\right)^2}{1 - \left(\dfrac{V_S}{V_P}\right)^2} \qquad (III)$$

Module de Young dynamique :

$$E_{dyn} = densité \times \frac{V_P \left(1 + v_{dyn}\right) \cdot \left(1 - 2 \cdot v_{dyn}\right)}{\left(1 - v_{dyn}\right)} \qquad (IV)$$

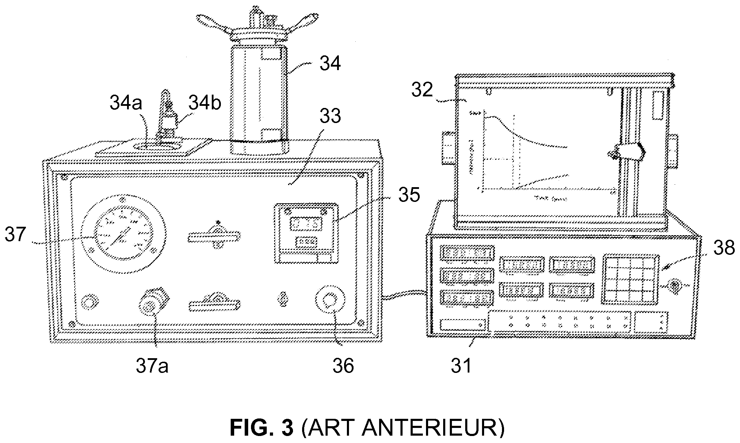

La figure 3 illustre un appareil de mesure de type UCA, tel que décrit dans le brevet US 4,259,868. Un tel dispositif de mesure comprend une cellule de mesure 34 destinée à recevoir le coulis de ciment et à être mise sous pression. Cette cellule 34 est mise sous pression dans un autoclave 33 par le biais de son insertion dans une ouverture 34a et d'une connexion avec un conduit de mise sous pression 34b. La pression et la température de l'autoclave, transmises à la cellule 34, sont contrôlées par des variateurs (37a, 36) et des écrans de contrôle (37, 35). Des transducteurs ultrasoniques (non représentés) sont couplés à la cellule de mesure 34, et permettent l'émission et la réception d'ondes ultrasoniques traversant l'échantillon du système cimentaire. Un ordinateur de contrôle 31 est relié à l'autoclave et comporte un clavier 38 permettant l'entrée de données et les fonctions de commande dans le dispositif de mesure, et un traceur numérique 32 permettant de visualiser la vitesse des ondes mesurées ainsi que la résistance à la compression uniaxiale calculée en temps réel.

Le protocole de mesure selon une méthode de type UCA ou MPRO suit le schéma suivant :

- préparation d'un volume de laitier de ciment ;
- remplissage de la cellule de mesure ;
- mise en conditions de l'échantillon du système cimentaire en appliquant une rampe d'augmentation de pression et de température pour atteindre les conditions in situ ($P_1$, $T_1$). Une fois ces conditions atteintes, la pression et la température restent constantes ;
- mesure, en fonction du temps, de :

  ◦ la vitesse des ondes de compression, et de vitesse des ondes de cisaillement dans le cas d'une mesure de type MPRO ;
  ◦ de la pression ; et
  ◦ de la température ;

  et utilisation d'une fonction de corrélation pour évaluer la résistance à la compression uniaxiale du système cimentaire en fonction du temps, et utilisation des relations *(III)* et *(IV)* décrites ci-dessus, pour évaluer le coefficient de poisson dynamique et le module de Young dynamique en fonction du temps dans le cas d'une mesure MPRO ;

- une fois l'essai terminé, démontage de l'éprouvette et observation de l'échantillon de ciment.

Il est entendu que l'invention ne se limite pas à la mesure de Vp(t) selon les méthodes de type UCA et MPRO décrites ici, et que l'homme du métier pourra choisir toute méthode permettant la mesure de la vitesse des ondes de compression d'un système cimentaire. Des exemples de telles méthodes sont par exemple décrits dans les documents suivants : US5859811, US5763773, US5357481, US5168470, US5001676, US4813028, US4779236, US4255798, US3401773, US2538114. Il est également évident que le procédé selon l'invention peut être mis en oeuvre sans avoir à effectuer cette étape de mesure de Vp(t), si cette donnée est connue par ailleurs pour le système cimentaire étudié.

Un avantage à utiliser la mesure de la vitesse des ondes de compression est que celle-ci constitue une mesure simple et normalisable, contribuant de ce fait à fournir un procédé facilement industrialisable. Le protocole expérimental utilisé pour déterminer au besoin Vp(t) est simple. Seule la connaissance de la vitesse des ondes de compression est nécessaire pour déterminer le comportement mécanique d'un ciment selon le procédé objet de la présente invention.

*Etape A*

L'étape A consiste à déterminer le degré d'hydratation du système cimentaire en fonction du temps α(t), à partir de la connaissance de la vitesse des ondes de compression Vp(t) du système cimentaire en question, mesurée dans un échantillon du système cimentaire, à une pression $P_1$ et une température $T_1$.

Pour cela, le procédé selon l'invention utilise une corrélation empirique 110 qui relie α(t) à $V_p$(t).

Selon un mode de réalisation de l'invention, le degré d'hydratation du système cimentaire en fonction du temps α(t) est calculé à partir de Vp(t) selon une relation linéaire. De manière préférée, le degré d'hydratation du système cimentaire en fonction du temps α(t) est calculé à partir de Vp(t) selon la relation linéaire suivante :

$$\alpha = \frac{(V_P - V_0)}{(V_\infty - V_0)} \qquad\qquad (V)$$

avec $V_0$ et $V_P$ correspondant respectivement à la vitesse des ondes de compression mesurée dans l'échantillon du système cimentaire au temps $t = 0$ et au temps t, et $V_\infty$ correspondant à la vitesse des ondes de compression dans l'échantillon du système cimentaire complètement hydraté.

Les valeurs de $V_p$ et $V_0$ sont connues au début de l'étape A. La valeur de $V_\infty$ peut être connue expérimentalement avec la même mesure que celle permettant de connaître Vp(t), et correspond à un échantillon dans lequel l'hydratation est complète. Connaissant Vp(t), $V_\infty$ est par exemple calculé à l'aide d'une régression linéaire.

Selon un mode de réalisation de l'invention, la valeur de $V_\infty$ est issue d'un modèle établi à partir de données expérimentales préliminaires issues d'essais de type UCA, permettant de prédire cette valeur pour tout système cimentaire. Selon ce mode de réalisation, il n'est pas nécessaire de réaliser une mesure de $V_\infty$ pour le système cimentaire objet du procédé selon l'invention.

Selon un mode de réalisation de l'invention, $V_\infty$ est égal à environ 3980 m/s.

*Etape B*

Le procédé selon l'invention comprend une étape B consistant à déterminer le degré d'hydratation $\alpha(t)$, issu de l'étape A, en fonction de valeurs souhaitées de finesse $\Phi_n$ du système cimentaire, de pression $P_n$ et/ou de température $T_n$.

Cette détermination utilise un modèle cinétique 120 de l'hydratation du système cimentaire. Selon un mode préféré de l'invention, ce modèle cinétique 120 comprend deux stades correspondant à deux sous-étapes de l'étape B : B-i et B-ii.

Selon un mode de réalisation de l'invention, le processus d'hydratation comprend un premier stade où l'hydratation est gouvernée principalement par un processus de nucléation et de croissance, et un deuxième stade où l'hydratation est gouvernée principalement par un processus diffusion des ions, ledit deuxième stade commençant lorsque le degré d'hydratation $\alpha$ atteint une valeur de degré d'hydratation seuil $\alpha^*$, cette valeur seuil $\alpha^*$ étant fonction de la température. L'étape B comprend les sous-étapes suivantes :

- (B-i) la détermination du degré d'hydratation $\alpha(t)$ pendant le premier stade du processus d'hydratation du système cimentaire;
- (B-ii) la détermination du degré d'hydratation $\alpha(t)$ pendant le deuxième stade du processus d'hydratation du système cimentaire.

Chacune des sous-étapes B-i et B-ii prend en compte la finesse $\Phi$ du système cimentaire, la pression et la température pour la détermination du degré d'hydratation $\alpha(t)$.

Le procédé prend donc avantageusement en compte les effets de la température et de la pression pour simuler l'évolution du degré d'hydratation du système cimentaire.

Selon un mode de réalisation de l'invention, la valeur de degré d'hydratation seuil $\alpha^*$ est évaluée en minimisant l'écart entre $\alpha(t)$, déterminé en utilisant le modèle cinétique 120, et $\alpha(t)$, déterminé expérimentalement à partir de la vitesse des ondes de compression, pour différentes températures, et à une pression constante, de manière à prendre en compte la variation de $\alpha^*$ en fonction de la température dans l'étape B. Avantageusement, la détermination expérimentale de $\alpha(t)$ est effectuée à différentes températures, et à une pression constante inférieure ou égale à 1 MPa, de préférence inférieure ou égale à 0.5 MPa, encore plus préférentiellement inférieure ou égale à 0.3 MPa. A de telles pressions, il peut être considéré que l'effet du volume d'activation est négligeable.

De manière préférée :

- le système cimentaire de composition initiale $C_0$ comprend un ciment et de l'eau, le ciment comprenant au moins une phase initiale réactive X ;
- le degré d'hydratation $\alpha(t)$ déterminé à l'étape B correspond à la moyenne pondérée des degrés d'hydratation de chacune des phases initiales réactives X du ciment ;
- le degré d'hydratation de chacune des phases initiales réactives X du ciment est fonction du rapport entre l'affinité chimique $A_X(\alpha)$ de la phase initiale réactive X, cette affinité chimique $A_X(\alpha)$ contrôlant le taux d'évolution de l'hydratation de la phase initiale réactive X, et le temps caractéristique associé à la réaction de la phase initiale réactive X avec l'eau $\tau_X$ ;
- et le temps caractéristique associé à la réaction de la phase initiale réactive X avec l'eau $\tau_x$ est fonction de la finesse Φ du ciment, de la pression et de la température.

Avantageusement, le temps caractéristique associé à la réaction de la phase initiale réactive X avec l'eau $\tau_x$ s'exprime selon l'équation suivante :

$$\tau_x\left(T,\Phi\right) = \sqrt[n_x+1]{\frac{\Phi_0}{\Phi}} \times \tau_x\left(T_0,\Phi_0\right)\exp\left(\frac{\Delta E_x}{R}\left(\frac{1}{T}-\frac{1}{T_0}\right)+\frac{\Delta V_x}{R}\left(\frac{P}{T}-\frac{P_0}{T_0}\right)\right) \qquad (VI)$$

avec $\Phi_0$ la finesse d'un ciment de référence, de préférence $\Phi_0 = 3600$ cm$^2$/g, $T_0$ la température initiale et $P_0$ la pression initiales au temps t=0 du processus d'hydratation, R la constante de gaz parfaits, $\Delta E_x$ l'énergie d'activation, $\Delta V_x$ le volume d'activation pour la nucléation et la croissance des hydrates lors du premier stade du processus d'hydratation, $n_x$ une constante, déterminée expérimentalement dont les valeurs sont publiées dans la littérature (Bernard et al.[12]).

*Sous-étape B-i*

Lors de la sous-étape B-i, on considère le premier stade d'évolution du degré d'hydratation, où l'hydratation est gouvernée principalement par un processus de nucléation et de croissance, tel que par exemple décrit par Avrami[3]. Ce premier stade comprend les phases d'accélération et de ralentissement du processus d'hydratation dans le cas d'un ciment Portland. Cette sous-étape B-i consiste à déterminer le degré d'hydratation du système cimentaire à partir des degrés d'hydratation de chacune des phases initiales réactives X du ciment selon la relation suivante :

$$\alpha = \sum_{x=1}^{N} m^{x'} \cdot \alpha_x \qquad (VII)$$

où N est le nombre de phases initiales réactives X. Par exemple, pour le clinker, X serait un des quatre constituants principaux du clinker ($C_3S$, $C_2S$, $C_3A$, $C_4AF$). $m^x$, est la fraction massique du constituant X.

Le degré d'hydratation de la phase initiale réactive $\alpha_x$ est défini comme le rapport entre la quantité de cette phase ayant réagie et la quantité initiale de cette phase, et vérifie l'équation suivante:

$$\frac{d\alpha_x}{dt} = \frac{A_x\left(\alpha\right)}{\tau_x} \qquad (VIII)$$

L'affinité chimique $A_x(\alpha)$ de la phase initiale réactive X vérifie l'équation suivante :

$$A_x\left(\alpha\right) = \left(1-\left(\alpha-\alpha_{0x}\right)\right)\cdot\left[-\ln\left(1-\left(\alpha-\alpha_{0x}\right)\right)\right]^{1-\frac{1}{n_x+1}} \qquad (IX)$$

où $n_x$ et $\alpha_{0x}$ sont des constantes déterminées expérimentalement et connues par l'homme de l'art (Bernard et al.[12]).

Le temps caractéristique associé à la réaction de la phase initiale réactive X avec l'eau $\tau_x$ est fonction de la finesse $\Phi$ du ciment, de la pression et de la température. Au cours de la sous-étape B-i, $\tau_x$ vérifie l'équation suivante :

$$\tau_x\left(T,\Phi\right) = \sqrt[n_x+1]{\frac{\Phi_0}{\Phi}} \times \tau_x\left(T_0,\Phi_0\right)\exp\left(\frac{\Delta E_x}{R}\left(\frac{1}{T}-\frac{1}{T_0}\right)+\frac{\Delta V_x}{R}\left(\frac{P}{T}-\frac{P_0}{T_0}\right)\right) \qquad (VI)$$

Dans laquelle $\Phi_0$ est la finesse d'un ciment de référence, de préférence $\Phi_0$=3600cm$^2$/g, $T_0$ la température initiale et $P_0$ la pression initiales au temps t=0 du processus d'hydratation, R la constante de gaz parfaits, égale à 8,314 J°K-1mol-1, $\Delta E_x$ l'énergie d'activation, $\Delta V_x$ le volume d'activation pour la nucléation et la croissance des hydrates lors des phases de croissance et de ralentissement du processus d'hydratation, égal à -27$\times$10$^{-6}$ m$^3$/mol, et $n_x$ est une constante déterminée expérimentalement et connue par l'homme de l'art (Bernard et al.[12]).

Les effets de la finesse du système cimentaire, de la pression et de la température sur la cinétique d'hydratation de ce dernier sont intégrés dans cette équation (*VI*). Cette équation établit que la vitesse d'évolution d'hydratation d'un système cimentaire croît avec la finesse, la pression et la température.

*Sous-étape B-ii*

Lors de la sous-étape B-ii, on considère le deuxième stade d'évolution du degré d'hydratation, où l'hydratation est gouvernée principalement par un processus de diffusion des ions. Ce deuxième stade comprend la phase de cure dans le cas d'un ciment Portland.

On appelle r* la valeur du rayon du grain de ciment entouré d'une couche d'hydrates lorsque le degré d'hydratation du ciment atteint une valeur de seuil $\alpha$*. La valeur du rayon diminue avec l'avancement de l'hydratation, et son taux de diminution peut être écrit en fonction d'une constante de diffusion D ($cm^2$/h) selon l'équation suivante :

$$-\frac{dr}{dt} = \frac{D}{r*-r} \qquad (X)$$

Comme précédemment, on peut exprimer le degré d'hydratation $\alpha(t)$ en fonction de l'affinité chimique $A(\alpha)$. $\alpha(t)$ et $A(\alpha)$ vérifient l'équation suivante :

$$\frac{d\alpha}{dt} = \frac{A(\alpha)}{\tau} \qquad (XI)$$

L'affinité chimique vérifie l'équation suivante :

$$A(\alpha) = \frac{(1-\alpha)^{2/3}}{(1-\alpha*)^{1/3} - (1-\alpha)^{1/3}} \qquad (XII)$$

Comme précédemment, on peut exprimer le temps caractéristique $\tau_x$ associé à la réaction de la phase initiale réactive X avec l'eau en fonction de la finesse $\Phi$ du ciment, de la pression et de la température. $\tau_x$ vérifie l'équation (*VI*) précédemment explicitée.

Au cours de ce deuxième stade, le temps caractéristique de référence $\tau_x(T_0,\Phi_0)$ est contrôlé par la diffusion des ions à travers la couche des hydrates autour des grains de ciment (Bernard et al.[12]) :

$$\tau_x(T_0, \Phi_0) = \frac{R^2}{3D} \qquad (XIII)$$

Le modèle cinétique 120 utilisé dans le procédé selon l'invention prend avantageusement en compte l'effet de la finesse du système cimentaire, de la température et de la pression pour simuler les résultats de l'évolution du degré d'hydratation du système cimentaire. Le volume d'activation $\Delta V_x$ est avantageusement considéré comme étant identique pour chacune des phases initiales réactives. La valeur de degré d'hydratation seuil $\alpha$* est évaluée en minimisant l'écart entre $\alpha(t)$, déterminé selon l'étape A, et $\alpha(t)$, déterminé expérimentalement pour le système cimentaire, pour différentes températures, et à une pression constante. Avantageusement, la pression est inférieure ou égale à 1 MPa, de préférence inférieure ou égale à 0.5 MPa, encore plus préférentiellement inférieure ou égale à 0.3 MPa. A cette faible pression, il est en effet possible de négliger l'effet du volume d'activation.

Selon un mode de réalisation de l'invention, la pression n'affecte pas le degré d'hydratation seuil $\alpha$*, qui varie seulement en fonction de la température. En particulier, au-delà d'une certaine valeur de la température d'hydratation, la phase de diffusion commence plus tôt à une température d'hydratation plus élevée, et la température plus élevée provoque l'augmentation du taux de C-S-H HD à long terme.

*Etape C*

Cette étape consiste à déterminer la composition du système cimentaire en fonction du temps C(t) et en fonction de valeurs souhaitées de finesse $\Phi_n$ du système cimentaire, de pression $P_n$, et/ou de température $T_n$, à partir du degré d'hydratation $\alpha(t)$ déterminé à l'étape B.

Pour cela, le procédé selon l'invention utilise un modèle compositionnel 130, qui permet de connaitre, à tout degré d'hydratation du système cimentaire, la teneur du système en chaque phase considérée. En particulier, la fraction volumique, massique ou molaire, de préférence volumique, de chaque phase du système cimentaire est déterminée.

En premier lieu, la connaissance de $\alpha(t)$, pour des valeurs souhaitées de finesse du système cimentaire, de pression et/ou de température permet d'estimer la fraction, de préférence volumique, des phases initiales du système cimentaire (phases réactives initiales du ciment et eau initiale consommée), en fonction du temps. Dans un second temps, à partir des relations entre la proportion molaire des réactants et des hydrates des réactions chimiques de l'hydratation du système cimentaire, la fraction, par exemple volumique, des phases hydratées du système cimentaire est calculée à un temps donné. En effet, les équations chimiques permettent de déterminer la proportion en nombres de moles des réactants et des hydrates. La fraction volumique est alors déterminée en connaissant la densité et la masse molaire des hydrates. Ainsi, les fractions, de préférence volumiques, des phases hydratées du système cimentaire, en particulier les phases C-S-H et de CH, sont déterminées. Enfin, la fraction, de préférence volumique, des aluminates est déterminée comme étant la différence entre la proportion initiale, de préférence le volume total initial, et la proportion calculée, de préférence le volume calculé, de toutes les autres phases.

Avantageusement, le modèle compositionnel 130 prend en compte la température à laquelle s'effectue l'hydratation, notamment en ce qui concerne la détermination des fractions, de préférence volumiques, des phases hydratées, en particulier de la phase C-S-H.

Lors de cette étape, la composition du système cimentaire C(t) est déterminée en fonction du temps, pour des valeurs souhaitées de finesse $\Phi_n$ du système cimentaire, de pression $P_n$ et/ou de température $T_n$.

Dans le procédé selon l'invention, le système cimentaire comprend m phases, parmi lesquelles :

- au moins une phase initiale réactive X, de préférence choisie dans le groupe constitué par le silicate tricalcique $C_3S$, le silicate dicalcique $C_2S$, l'aluminate tricalcique $C_3A$, l'aluminoferrite tétracalcique $C_4F$, et leurs associations ;
- au moins une phase hydratée Y issue de l'hydratation d'au moins une phase initiale réactive X, de préférence choisie dans le groupe constitué par le silicate de calcium hydraté C-S-H, l'hydroxyde de calcium CH, le trisulfoaluminate de calcium hydraté TSA, le monosulfate de calcium hydraté AFm, l'aluminoferrite de calcium hydraté, et leurs associations ;
- de l'eau;

la composition initiale du système cimentaire $C_0$ comprenant un volume initial d'eau déterminé $V_w^0$ et au moins une phase initiale réactive X.

L'étape C comprend l'estimation de la composition du système cimentaire en fonction du temps C(t) par la détermination de la fraction molaire, massique ou volumique des *m* phases du système cimentaire.

Selon un mode de réalisation de l'invention :

- le volume d'au moins une phase initiale réactive X en fonction du temps $V_x(t)$ est calculé selon l'équation *(XIV)* :

$$V_x\left(t\right) = V_x^0\left(1 - \alpha_x\left(t\right)\right) \qquad (XIV)$$

avec $V_x^0$ le volume initial de la phase initiale réactive X, et $\alpha_x(t)$ le degré d'hydratation de la phase initiale réactive X en fonction du temps ;

- le volume d'au moins une phase hydratée en fonction du temps est calculé selon l'équation *(XV)* :

$$V\left(t\right) = \sum V_y^x \cdot \alpha_x\left(t\right) \qquad (XV)$$

avec $V_y^x$ le volume occupé par la phase hydratée *Y* formée par la phase réactive *X* et $\alpha_x(t)$ le degré d'hydratation de la phase réactive *X* en fonction du temps ;

- le volume de l'eau en fonction du temps $V_w(t)$ est calculé selon l'équation *(XVI)* :

$$V_w\left(t\right) = V_w^0 - \sum V_w^x \cdot \alpha_x\left(t\right) \qquad (XVI)$$

avec $V_w^0$ le volume initial d'eau dans le système cimentaire, $V_w^x$ le volume de l'eau consommée par la phase X, $\alpha_x(t)$ le degré d'hydratation de X en fonction du temps.

Le procédé selon l'invention peut en outre prendre en compte d'autres phases, telle que de l'eau entrante, diffusant du milieu environnemental vers les pores du système cimentaire, ou au moins une phase gazeuse. Dans ce cas, le procédé permet en outre de déterminer une fraction, par exemple volumique, d'eau entrante dans le système cimentaire en fonction du temps, et une fraction, par exemple volumique, de gaz en fonction du temps. Pour cela, les fractions de l'eau entrante et/ou des phases gazeuses sont déterminées en se basant sur le phénomène de retrait chimique se produisant lors de l'hydratation du système cimentaire.

Selon un mode de réalisation de l'invention, le volume de la phase hydratée C-S-H est déterminé en fonction de la température à laquelle se produit l'hydratation. En particulier, la fraction des phases C-S-H LD et C-S-H HD est déterminée différemment selon que l'hydratation s'effectue à température ambiante (20°C$\pm$5°C), ou à température supérieure ou inférieure à la température ambiante.

Dans le cas où l'hydratation se produit à température ambiante, les fractions, de préférence volumiques, de C-S-H LD et C-S-H HD peuvent s'exprimer selon les équations suivantes (Bernard et al.[12]) :

$$V_{LD}(t) = \sum V_{C-S-H}^i \left( \alpha * - \left( \alpha * - \alpha^i(t) \right) \times \mathrm{H}\left[ \alpha * - \alpha^i(t) \right] \right) \qquad (XVII)$$

$$V_{HD}(t) = \sum V_{C-S-H}^i \left( \alpha^i(t) - \alpha * \right) \times \mathrm{H}\left[ \alpha^i(t) - \alpha * \right] \qquad (XVIII)$$

avec H la fonction de Heaviside, et i désigne chacune des réactions ayant la phase hydratée comme produit de la réaction.

La densité de C-S-H HD et C-S-H LD est exprimée par:

$$\rho_{HD} = \left( 1 - \phi_{HD} \right) \cdot \rho_g + \phi_{HD} \cdot \rho_W \qquad (XIX)$$

$$\rho_{LD} = \left( 1 - \phi_{LD} \right) \cdot \rho_g + \phi_{LD} \cdot \rho_W \qquad (XX)$$

avec $\rho_g$ la densité du globule, de préférence égale à 2,65 g/cm$^3$, $\rho_w$ la densité de l'eau, égale à 1 g/cm$^3$), et $\phi_{HD}$ et $\phi_{LD}$ les porosités respectives de C-S-H HD et C-S-H LD.

Dans le cas où l'hydratation se produit à une température supérieure à la température ambiante, la détermination de la composition C(t) prend en compte l'influence de la température, via le degré d'hydratation seuil $\alpha*$ dépendant de la température, sur les fractions de C-S-H LD et C-S-H HD, et éventuellement sur la fraction de l'eau entrante. Les fractions, de préférence volumiques, de C-S-H LD et C-S-H HD peuvent s'exprimer par les mêmes équations (XVII) et (XVIII) ci-dessus, qui prennent en compte une valeur de la porosité de la phase C-S-H HD différente de celle à température ambiante. La détermination de la porosité du C-S-H HD à une température donnée est obtenue en supposant que 1) la quantité de globules (C-S-H LD et de C-S-H HD) ne dépend que du degré d'hydratation, 2) le volume des globules est constant pour une température d'hydratation donnée, 3) la densité des globules de C-S-H n'est quasiment pas modifiée par la température, 4) la porosité totale n'est pas modifiée par la température et 5) le volume total des pores mesuré par passage à l'étuve à 105°C est considéré comme étant la somme des volumes des pores provenant des globules, du C-S-H LD, du C-S-H HD et du volume des capillaires.

En effet, dans le cas d'une température supérieure à la température ambiante, la fraction volumique de C-S-H HD et sa densité sont plus importantes (porosité de C-S-H HD plus faible) et la fraction volumique de C-S-H LD est plus faible qu'à température ambiante. La macroporosité du système cimentaire augmente également avec une température plus élevée, pour un même degré d'hydratation.

On obtient ainsi un système d'équation (XXI) permettant de calculer les fractions volumiques en C-S-H LD et C-S-H HD ainsi que la porosité du C-S-H HD à une température $T_2$ en fonction de ces mêmes paramètres à une température $T_1$, sous couvert que la différence de porosité capillaire soit connue.

$$\frac{f_{LD2}}{\alpha_2^*\left(\dfrac{1-\alpha_2^*}{1-\phi_{HD2}}+\dfrac{\alpha_2^*}{1-\phi_{LD}}\right)}=\frac{f_{LD1}}{\alpha_1^*\left(\dfrac{1-\alpha_1^*}{1-\phi_{HD1}}+\dfrac{\alpha_1^*}{1-\phi_{LD}}\right)}$$

$$\frac{f_{HD2}}{\left(1-\alpha_2^*\right)\left(\dfrac{1-\alpha_2^*}{1-\phi_{HD2}}+\dfrac{\alpha_2^*}{1-\phi_{LD}}\right)}=\frac{f_{HD1}}{\left(1-\alpha_1^*\right)\left(\dfrac{1-\alpha_1^*}{1-\phi_{HD1}}+\dfrac{\alpha_1^*}{1-\phi_{LD}}\right)} \qquad (XXI)$$

$$f_{LD2}\phi_{LD}+f_{HD2}\phi_{HD2}-f_{LD1}\phi_{LD}-f_{HD1}\phi_{HD1}=\phi_{cp1}-\phi_{cp2}$$

La différence de porosité capillaire est par exemple prise égale à 6% entre le cas à température de 60°C et celui à température de 20°C, basé sur des mesures au porosimètre à mercure. Ainsi, les porosités de C-S-H HD et de C-S-H LD qui sont par exemple respectivement de 24% et 37% à une température ambiante de 20°C, sont respectivement de 17% et 37% à une température de à 60°C.

*Etape D*

Cette étape consiste à déterminer au moins un paramètre mécanique du système cimentaire en fonction du temps, et en fonction des valeurs souhaitées de finesse $\Phi_n$ du système cimentaire, de pression $P_n$ et/ou de température $T_n$, à partir de la composition du système cimentaire C(t) déterminée à l'étape C.

Pour cela, le procédé selon l'invention utilise une méthode d'analyse multi-compositionnelle 140, basée sur des techniques d'homogénéisation telles qu'utilisées en micromécanique. Cette méthode d'analyse multi-compositionnelle 140 prend en compte un modèle multi-échelle du système cimentaire pour déterminer, à partir de techniques d'homogénéisation, au moins un paramètre mécanique du système cimentaire en fonction du temps, pour une valeur donnée de finesse du système cimentaire, de pression et/ou de température, connaissant la composition du système cimentaire C(t) déterminée à l'étape C, et connaissant l'évolution des composants du ciment du système cimentaire obtenue à partir du modèle de la cinétique d'hydratation utilisé à l'étape A. Le modèle multi-échelle comprend au moins une échelle élémentaire représentant des globules de la phase solide C-S-H et une échelle macroscopique du système cimentaire, de préférence un modèle multi-échelle comprenant trois échelles.

Lors de cette étape D, l'hétérogénéité physico-chimique du système cimentaire, en particulier du ciment durci, est représentée par un modèle multi-échelle. Une échelle locale comprend des unités élémentaires appelées globules qui représentant la phase hydratée solide C-S-H. Cette échelle locale représente la plus petite échelle du modèle multi-échelle utilisé lors de cette étape. Une échelle macroscopique représente le système cimentaire à l'échelle macroscopique. Des échelles intermédiaires peuvent être utilisées. Le module d'incompressibilité $k_s$ et le module de cisaillement $g_s$ des globules constituent les deux inconnues du modèle multi-échelle.

Classiquement, la micromécanique, en mécanique des matériaux, consiste à estimer les propriétés mécaniques d'un matériau en considérant un problème aux limites d'un volume élémentaire représentatif (VER). Selon Zaoui[14], il y a trois étapes pour l'homogénéisation : description, localisation et homogénéisation.

Dans la première étape, le VER doit être bien choisi pour représenter le comportement macroscopique du matériau. Ainsi, pour incorporer toute l'information mécanique et géométrique des phases hétérogènes du matériau, le VER est de préférence suffisamment grand. De plus, dans le contexte des milieux continus, ce volume est de préférence suffisamment petit pour décrire la continuité de la structure macroscopique.

Dans la deuxième étape, l'interaction des phases dans le VER est considérée. Différents schémas d'homogénéisation bien connus sont utilisés. Le schéma « dilué » ne considère aucune interaction entre les inclusions dans le VER, et ne peut être utilisé que pour des fractions volumiques faibles des inclusions. Le schéma « Mori-Tanaka », qui prend en compte l'interaction entre les inclusions, est couramment utilisé pour une microstructure de composites à matrice et inclusions. La différence entre le schéma « Mori-Tanaka » et le schéma « auto-cohérent » est que ce dernier prend le matériau homogénéisé comme la matrice en résolvant un système des équations non-linéaires pour l'estimation des propriétés mécaniques effectives du matériau. Pour le cas d'un matériau composite à matrice et inclusions, le schéma plus avancé de « Ponte-Castaneda et Willis » (Pont Castenada et al.[16]) prend en outre en compte l'information de l'orientation et de la distribution spatiale des inclusions dans le VER. Après avoir choisi le schéma d'estimation, les relations entre les tenseurs locaux de déformations et de contraintes et les tenseurs macroscopiques de déformations et de contraintes sont établies dans cette étape. Ces relations forment un tenseur appelé « tenseur de localisation des déformations ». Dans les cas où les conditions aux limites sont homogènes, le lemme de Hill[17] permet de démontrer que la déformation et la contrainte macroscopiques sont respectivement égales à la moyenne du champ des déformations et des contraintes microscopiques respectivement. Pour des inclusions ellipsoïdales élastiques linéaires homogènes plongées dans un milieu solide élastique linéaire homogène, Eshelby[18] a démontré que la déformation dans les inclusions est homogène. Enfin, le tenseur de localisation des déformations est déterminé en fonction des caractéristiques de la matrice et des inclusions.

Dans l'étape d'homogénéisation, en combinant des équations du comportement du matériau au niveau local et macroscopique, les paramètres élastiques et de couplage hydro-mécanique peuvent être exprimés en fonction des tenseurs de localisation de déformations. Pour un cas drainé, le tenseur de la rigidité du matériau est exprimé par :

$$C^{\mathrm{hom}} = 3K_d^{\mathrm{hom}}\mathbf{J} + 2G^{\mathrm{hom}}\mathbf{K} \qquad\qquad (XXII)$$

où $\mathbf{J}_{ijkl} = \dfrac{\delta_{ij}\delta_{kl}}{3}$ ; $\mathbf{K} = \mathbf{I} - \mathbf{J}$ ; $\mathbf{I} = \dfrac{\delta_{ik}\delta_{jl} + \delta_{il}\delta_{jk}}{2}$ . $\delta_{ij}$ dénote le delta Kronecker, $K_d^{\mathrm{hom}}$ est le module d'incompressibilité drainé, $G^{\mathrm{hom}}$ est le module de cisaillement. Selon Zaoui[14], pour un cas isotrope avec $n$ inclusions sphériques, le module d'incompressibilité drainé et le module de cisaillement effectifs sont calculés par les expressions *(XXIII) et (XXIV)* suivantes :

$$K_d^{\mathrm{hom}} = \sum_{r=1}^{n} f_r k_r A_r^v \qquad\qquad (XXIII)$$

$$G^{\mathrm{hom}} = \sum_{r=1}^{n} f_r g_r A_r^d \qquad\qquad (XXIV)$$

dans lesquelles $f_r$, $k_r$, et $g_r$ sont respectivement la fraction volumique, le module d'incompressibilité et le module de cisaillement de la phase $r$. Pour le cas où toutes les inclusions sont sphériques, le tenseur de localisation de déformation volumique $A_v^r$ et le tenseur de localisation de déformation déviatorique $A_d^r$ sont données par les équations *(XXV) et (XXVI)* suivantes :

$$A_r^v = \frac{\left(1 + \alpha_0 (k_r / k_0 - 1)\right)^{-1}}{\sum_r f_r \left(1 + \alpha_0 (k_r / k_0 - 1)\right)^{-1}} \qquad\qquad (XXV)$$

$$A_r^d = \frac{\left(1 + \beta_0 (g_r / g_0 - 1)\right)^{-1}}{\sum_r f_r \left(1 + \beta_0 (g_r / g_0 - 1)\right)^{-1}} \qquad\qquad (XXVI)$$

avec $\alpha_0 = \dfrac{3k_0}{3k_0 + 4g_0}$ , $\beta_0 = \dfrac{6(k_0 + 2g_0)}{5(3k_0 + 4g_0)}$, $k_0$ le module d'incompressibilité de la matrice de référence, et $g_0$ le module de cisaillement de la matrice de référence.

Pour calculer le tenseur de rigidité de la phase solide, le tenseur de coefficient de Biot et le module Biot homogénéisés, deux cas sont considérés :

Dans le premier cas, le matériau considéré comprend une phase poreuse unique (les pores) et $n$-$1$ phase solide. D'après Ulm et al.[19], le tenseur de coefficient de Biot d'un matériau ayant une seule phase poreuse est donné par l'équation *(XXVII)* suivante:

$$b^{\mathrm{hom}} = 1 : \left( \mathbf{I} - \sum_{r=1}^{n-1} f_r \langle A \rangle_{V_r} \right) \qquad (XXVII)$$

Le signe $\langle A \rangle_V$ dénote la valeur moyenne volumique de A dans le volume V. Le module de Biot homogénéisé est écrit sous la forme *(XXVIII)* suivante :

$$\frac{1}{N^{\mathrm{hom}}} = 1 : \sum_{r=1}^{n-1} f_r c_r^{-1} : \left( 1 - 1 : \langle A \rangle_{V_r} \right) \qquad (XXVIII)$$

où $c_r$ est le tenseur de rigidité de la phase *r* qui est défini par la formule *(XXIX)* suivante :

$$c_r = 3k_r \mathbf{J} + 2g_r \mathbf{K} \qquad (XXIX)$$

Dans le deuxième cas, le matériau est un matériau poreux multi-échelle, par exemple deux échelles par souci de simplification. La généralisation à *N* échelles successives se fait alors sans difficulté. La configuration suivante est prise en compte : le matériau comprend *q* phases poreuses (solide et les petits pores), un volume de pore (larges pores) et *n-q-1* phases solides. L'homogénéisation consiste à réaliser deux étapes I et II : la première étape II consiste à homogénéiser séparément q phases poreuses pour obtenir les paramètres effectifs de chaque phase de l'étape I ( $c_r^I$, $b_r^I$, $N_r^I$, $c_{sr}^I$ qui sont respectivement le tenseur de rigidité, le tenseur de coefficient de Biot, le module de Biot, et le tenseur de rigidité de la phase solide de la phase *r* dans l'étape I) comme présenté dans le premier cas ci-dessus. La deuxième étape II consiste à homogénéiser *q* phases poreuses, un volume des larges pores et *n-q-1* phases solides. Le tenseur Biot homogénéisé dans la deuxième étape est exprimé par l'équation *(XXX)* suivante (Ulm et al.[19]) :

$$b^{\mathrm{hom}} = 1 - \sum_{r=1}^{n-1} \left( f_r \langle A \rangle_{V_r} : \left( 1 - b_r^I \right) \right) \qquad (XXX)$$

où $b_r$ est le coefficient de Biot de la phase *r*. Il faut noter que le coefficient de Biot des phases solides est égal à 0. Le module de Biot effectif est donné par l'expression *(XXXI)* suivante (Ulm et al.[19]) :

$$\frac{1}{N^{\mathrm{hom}}} = \sum_{r=1}^{n-1} f_r \left( \left( c_{sr}^I \right)^{-1} : \left( 1 - 1 : \langle A \rangle_{V_r} \right) : \left( 1 - b_r^I \right) + \frac{1}{N_r^I} \right) \qquad (XXXI)$$

Selon un mode de réalisation, l'hétérogénéité du système cimentaire à l'étape D se manifeste à trois échelles (Jennings [11]) :

- Une échelle élémentaire Ech.0 dans laquelle les globules ont une longueur caractéristique de l'ordre de $10^{-9}$m, et sont considérés comme représentant la phase solide de C-S-H.
- Une première échelle Ech.1 correspond aux phases C-S-H LD et C-S-H HD, sous forme de globules, ayant une longueur caractéristique comprise entre environ $10^{-9}$ - $10^{-8}$m et comprend en outre les pores de gel C-S-H.
- Une deuxième échelle Ech.2 correspond au système cimentaire ayant une longueur caractéristique supérieure à environ $10^{-8}$m et comprend les phases C-S-H LD, C-S-H HD, CH, le volume des pores capillaires, les aluminates et les phases initiales réactives (les grains du clinker).

Au sein de la première échelle Ech.1, deux inclusions sont considérées : d'une part la matrice formée par des globules solides, et d'autre part les pores dans les phases C-S-H LD et C-S-H HD. La matrice occupe par exemple 63% de volume pour le cas de la phase C-S-H LD, et elle occupe par exemple une proportion supérieure à 76%, dépendant de la température, de volume pour la phase C-S-H HD. Le schéma de Mori-Tanaka (Ghabezloo[13]) est choisi, avec la phase solide jouant le rôle le milieu de référence. Ce schéma de Mori-Tanaka est approprié pour les matériaux dont la phase solide est dominante et pour lesquels il y a des interactions entre les particules (Bernard et al[12]). Les paramètres mécaniques effectifs, par exemple le module d'incompressibilité et le module de cisaillement, de la phase C-S-H, sous ses deux formes C-S-H LD et C-S-H HD, peuvent être déterminées selon des équations *(XXXII)* et *(XXXIII)* suivantes, présentées dans les travaux de Ghabezloo[13, 20].

$$K_X^{\mathrm{hom}} = \left(1 - \phi_X\right) k_S A_{e,X}^v \qquad (XXXII)$$

$$G_X^{\mathrm{hom}} = \left(1 - \phi_X\right) g_S A_{e,X}^d \qquad (XXXIII)$$

Où X désigne la phase C-S-H LD ou la phase C-S-H HD, $k_s$ et $g_s$ sont respectivement le module d'incompressibilité et le module de cisaillement des globules qui représentent la phase hydratée solide C-S-H à l'échelle élémentaire Ech.0 (« s » comme solide).

En supposant une géométrie sphérique pour toutes les phases, les tenseurs de localisation de déformations sont calculés selon les équations *(XXV)* et *(XXVI)* décrites ci-dessus, avec $k_0 = k_s$ et $g_0 = g_s$ ($k_0$ est le module d'incompressibilité de la matrice de référence, et $g_0$ est le module de cisaillement de la matrice de référence).

Au sein de la deuxième échelle Ech. 2, l'évolution de la fraction volumique des phases du système cimentaire au cours de l'hydratation est prise en compte. Avantageusement, le schéma « auto-cohérent » est utilisé à cette échelle. Celui-ci permet de prendre en compte le seuil de percolation, et est approprié pour estimer les propriétés élastiques du système cimentaire à cette échelle (Ghabezloo[13]). Cette deuxième échelle Ech.2 comprend six phases : C-S-H LD (LD), C-S-H HD (HD), CH, le volume des pores capillaires (cp), des aluminates (AL), et les phases initiales réactives du ciment, qui sont par exemple les grains de clinker (CK). A cette échelle Ech.2, la phase des aluminates (phase solide) est supposée avoir des propriétés élastiques similaires à celles de C-S-H LD, qui ne sont pas modifiées par la température d'hydratation. Les expressions des modules élastiques homogénéisés sont les suivantes :

$$K_{cp}^{\mathrm{hom}} = \left(f_{LD} + f_{AL}\right) K_{LD}^{\mathrm{hom}} A_{LD,cp}^v + f_{HD} K_{HD}^{\mathrm{hom}} A_{HD,cp}^v + f_{CH} k_{CH} A_{CH,cp}^v + f_{CK} k_{CK} A_{CK,cp}^v \quad (XXXIV)$$

$$G_{cp}^{\mathrm{hom}} = \left(f_{LD} + f_{AL}\right) G_{LD}^{\mathrm{hom}} A_{LD,cp}^v + f_{HD} G_{HD}^{\mathrm{hom}} A_{HD,cp}^v + f_{CH} g_{CH} A_{CH,cp}^v + f_{CK} g_{CK} A_{CK,cp}^v \quad (XXXV)$$

Les tenseurs de localisation sont déterminés selon les équations *(XXV)* et *(XXVI)* décrites ci-dessus, avec $k_0 = K_{cp}^{\mathrm{hom}}$, et $g_0 = G_{cp}^{\mathrm{hom}}$ ($k_0$ est le module d'incompressibilité de la matrice de référence, et $g_0$ est le module de cisaillement de la matrice de référence).

Les paramètres à cette échelle Ech.2 sont par exemple les propriétés élastiques de CH et des phases initiales réactives du ciment (par exemple les quatre composants principaux du clinker).

*Programme informatique*

Selon un autre de ses aspects, l'invention concerne un produit programme informatique permettant de conserver dans une mémoire d'une unité d'un processeur ou sur un support de mémoire amovible approprié pour coopérer avec ladite unité du processeur, le produit-programme comprenant des instructions pour mettre en oeuvre le procédé selon l'invention

**EXEMPLES**

L'invention sera mise en évidence avec ses avantages apparents au travers de l'exemple ci-dessous, donné à titre non limitatif.

*Le système cimentaire cem1*

Le système cimentaire *cem1* donné en exemple ci-dessous comprend un ciment Portland de classe G, dont la composition est donnée dans le tableau 3.

**TABLEAU 3**

| Nom chimique | Formule chimique | Symbole | Pourcentage de masse (%) |
|---|---|---|---|
| Tricalcium silicate (Alite) | $3CaO.SiO_2$ | $C_3S$ | 55 |
| Dicalcium silicate (Belite) | $2CaO.SiO_2$ | $C_2S$ | 18 |
| Tricalcium aluminate | $3CaO.Al_2O_3$ | $C_3A$ | 10 |
| Tetracalcium aluminoferrite (Ferrite) | $4CaO.Al_2O_3 Fe_2O_3$ | $C_4AF$ | 8 |
| Calcium sulfate dihydrate (gypsum) | $CaSO_4.2H_2O$ | CSH2 | 6 |

La composition initiale $C_0$ du système cimentaire cem1 est donnée dans le tableau 4 ci-dessous. Elle correspond à un rapport eau sur ciment égal à environ 044 (w/c en anglais).

**TABLEAU 4**

| Composant | Quantité [gr] |
|---|---|
| Ciment | 914,12 |
| Eau | 396,13 |
| Agent anti-mousse (D047) | 7,31 |
| Dispersant (D604AM) | 11,06 |
| Agent anti-sédimentation (D153) | 1,37 |

La fabrication du laitier de ciment est effectuée de la manière suivante, par un mixage en 5 étapes:

- mélange de l'eau distillée avec un agent anti-sédimentation (D153);
- mixage pendant au moins 5 minutes avec une vitesse d'environ 4200 tours par minute ;
- introduction des deux autres adjuvants : un dispersant et un agent anti-mousse (D604AM et D47) ;
- malaxage à haute vitesse (une vitesse de 4200 tours par minute) pendant environ 15 secondes en ajoutant du ciment ;
- augmentation de la vitesse à 12000 tours par minute en mixant pendant 35 secondes.

*Test UCA sur un échantillon du système cimentaire cem1*

Le principe de la méthode et un dispositif de type UCA ont été décrits plus haut et s'appliquent ici. Après l'installation de la cellule UCA dans le système de génération de pression et de température, la pression est tout d'abord augmentée à la valeur souhaitée (par exemple 0,3 MPa, 20 MPa ou 40 MPa) et la température est ensuite augmentée jusqu'à température de l'essai en 30 mn. Les mesures de temps de transit ont été réalisées dès le début de l'essai, à t=0 et T=25°C.

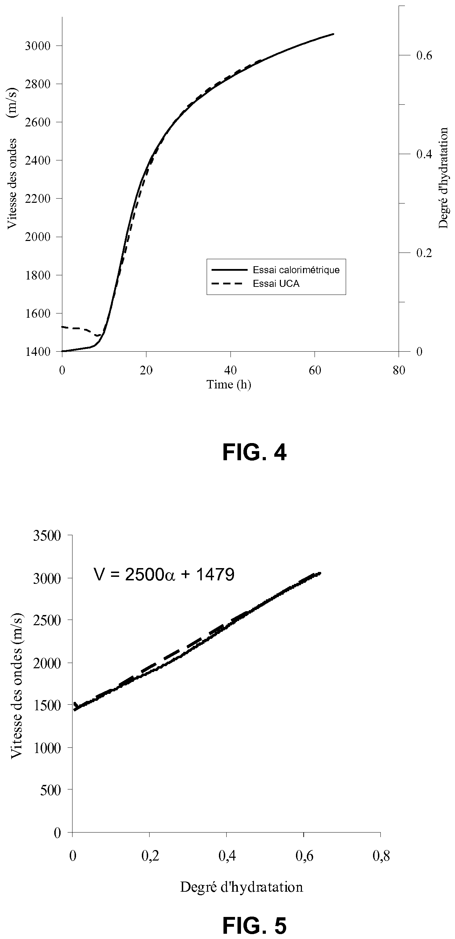

La figure 4 montre la vitesse des ondes de compression en fonction du temps Vp(t) obtenue.

*Essai calorimétrique sur un échantillon du système cimentaire cem1*

Le principe d'un essai calorimétrique a été décrit plus haut et s'applique ici. Le système cimentaire *cem1* est testé en réalisant un essai calorimétrique isotherme à 25°C.
La figure 4 montre le degré d'hydratation issu de cet essai calorimétrique. Les courbes $\alpha(t)$ et $V_p(t)$ se confondent presque complètement, ce qui montre qu'une relation linéaire existe entre l'évolution au cours du temps du degré d'hydratation $\alpha(t)$ du système cimentaire, mesuré par calorimétrie, et l'évolution au cours du temps de la vitesse des ondes de compression $V_p(t)$.
La figure 5 montre que la relation entre $\alpha(t)$ et $V_p(t)$ est quasi linéaire. La régression linéaire s'exprime ainsi : $V = 2500 \alpha + 1479$.

*Essais uniaxiaux sur un échantillon du système cimentaire cem1*

Des essais uniaxiaux permettant d'obtenir des mesures des paramètres mécaniques, en particulier de déformabilité, sont effectués sur le système *cem1.* Afin de mesurer l'effet de la température sur l'évolution des propriétés élastiques du système cimentaire en cours d'hydratation, les blocs cylindriques d'une longueur de 250 mm et d'un diamètre de 100 mm ont été curés à 20°C et 60°C à la pression atmosphérique dans l'eau saturée en chaux. Après deux jours de cure, ces blocs ont été carottés et sciés pour obtenir des échantillons cylindriques d'une longueur d'environ 100 mm et d'un diamètre d'environ 40 mm. Ces échantillons ont ensuite été conservés dans une solution neutre (pH=13). Des essais uniaxiaux ont été effectués aux âges suivants : 3 jours, 4 jours, 7 jours, 14 jours et 35 jours. Les essais uniaxiaux ont été effectués à l'aide d'une presse d'une capacité de 50 tonnes. La vitesse de déplacement uniaxial utilisée est de 0,3 $\mu$m/s. Trois capteurs LVDT (Linear variable Differential Transformer) ont été employés pour mesurer la déformation axiale. Le module de Young est évalué à partir de cycles de décharge - recharge de 0MPa à 18MPa de contrainte axiale.

*Etapes A et B du procédé : détermination de $\alpha(t)$ à $P_1$, $T_1$, et à différentes valeurs de finesse $\Phi_n$ du système cimentaire, de pression $P_n$ et de température $T_n$*

La vitesse des ondes de compression en fonction du temps $V_p(t)$ est mesurée dans un échantillon du système cimentaire *cem1,* à une pression $P_1$ et une température $T_1$, tel que décrit dans le test UCA ci-dessus.

$V\infty$ est déterminé à partir du test UCA, et correspond à 37979 m/s.

Connaissant $V_p(t)$ (voir figure 4), le degré d'hydratation en fonction du temps $\alpha(t)$ est déterminé en utilisant la relation linéaire $\alpha = (V_P - _0)/(V_\infty - V_0)$

Ce degré d'hydratation en fonction du temps $\alpha(t)$ est déterminé pour une pression $P_1$, et une température $T_1$, avec $P_1$ = 0.3 ou 20 MPa et $T_1$ = 25 °C.

La valeur de $\tau_x(T_0,\Phi_0)$ utilisée dans la formule *(VI)* dans le cas du premier stade du processus d'hydratation, gouverné principalement par les phénomènes de nucléation et de croissance, est calculée, en utilisant les valeurs des constantes connues par l'homme de l'art (Bernard et al.[12]). Ces valeurs sont reprises dans le tableau 5. Pour chaque phase initiale réactive ($C_3S$, $C_2S$, $C_3A$, $C_4AF$), l'affinité chimique est calculée en utilisant les valeurs du tableau 5.

**TABLEAU 5**

| Clinker | $\tau_x(T_0,\Phi)$ | $n_x$+1 | $\alpha_{x0}$ | $\Delta E_x/R$ (°K) |
|---|---|---|---|---|
| $C_3S$ | 13,24 | 1,76 | 0,02 | 4800 |
| $C_2S$ | 72,01 | 1,00 | 0 | 2500 |
| $C_3A$ | 59 | 0,90 | 0,04 | 5500 |
| $C_4AF$ | 24,68 | 2,34 | 0,4 | 4200 |

La valeur de $\tau_x(T_0,\Phi_0)$ utilisée dans la formule *(VI)* dans le cas du deuxième stade du processus d'hydratation du système cimentaire, gouverné principalement par le phénomène de diffusion (période de cure dans le cas de *cem1*), est calculée à partir des valeurs données dans le tableau 6 suivant :

**TABLEAU 6**

| Clinker | $C_3S$ | $C_2S$ | $C_3A$ | $C_4AF$ |
|---|---|---|---|---|
| $\tau_x$ (h) | 3492 | 564939 | 1439 | 3433 |

La valeur du degré d'hydratation seuil $\alpha^*$ pour le système cimentaire *cem1* est donnée dans le tableau 7 ci-dessous. $\alpha^*$ est évalué en minimisant l'écart entre les courbes déterminées à partir du procédé selon l'invention, et les courbes expérimentales pour différentes températures sous 0,3MPa car, pour cette faible pression, on peut négliger l'effet de la variable $\Delta V$ exprimée dans la formule *(VI)*.

**TABLEAU 7**

| T (°C) | 7 | 13 | 15 | 25 | 30 | | 40 | | 60 | |
|---|---|---|---|---|---|---|---|---|---|---|
| Pression (MPa) | 0,3 | 0,3 | 40 | 0,3 | 20 | 40 | 0,3 | 40 | 0,3 | 20 |
| $\alpha^*$ | 0,68 | 0,70 | 0,68 | 0,72 | 0,72 | 0,72 | 0,60 | 0,60 | 0,40 | 0,40 |

Les figures 6 à 8, qui comparent des données expérimentales issues d'essais UCA tels que décrit plus haut, et les données issues de la détermination du degré d'hydratation selon les étapes A et B du procédé objet de l'invention (courbes en pointillés, indiquée en tant que « simulation » dans la légende), montrent la bonne capacité prédictive des étapes A et B du procédé objet de l'invention.

*Etape C du procédé : détermination de la composition C(t))*

Dans cette étape, les stades 1 et 2 du processus d'hydratation, comprenant les phases d'hydratation initiale et dormante, ne sont pas pris en compte. Les stades 3 et 4, comprenant les phases d'accélération et de ralentissement de l'hydratation, sont pris en compte et simulées selon par la théorie de la nucléation et croissance d'Avrami[3]. Le stade 5 comprenant la phase de cure est également pris en compte, et est considéré comme étant contrôlé par des phénomènes de diffusion.

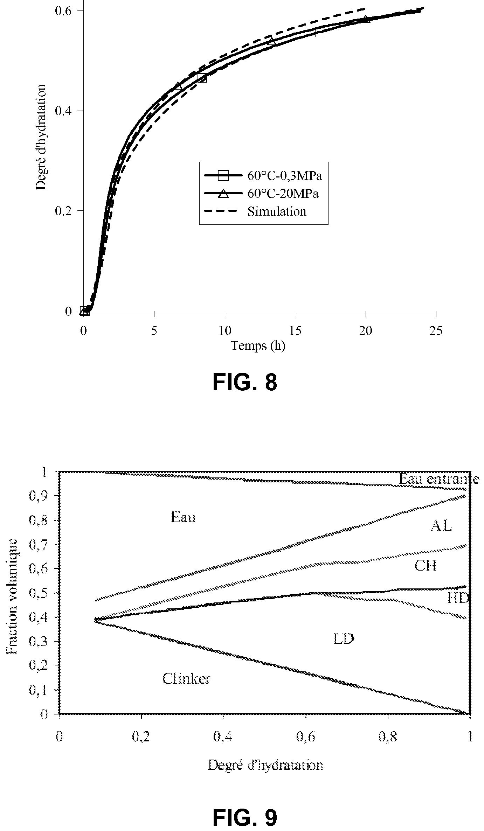

La figure 9 représente l'évolution des fractions volumiques des différentes phases du système cimentaire *cem1* en fonction du degré d'hydratation, pour une température d'hydratation de 20°C et à pression atmosphérique.

*Etape D du procédé*

Le tableau 8 présente les modules d'incompressibilité k et module de cisaillement g des différents composants solides pris en compte lors de l'analyse multi-échelle, selon l'étape D du procédé : les quatre phases du clinker du système *cem*1, la phase hydratée CH et les globules représentant la phase solide C-S-H du système cimentaire *cem1.* Toutes ces valeurs ont été obtenues à partir de la littérature, sauf en ce qui concerne les modules des globules qui ont été obtenus par calage avec les données d'essais réalisés par Bourissai[21].

L'analyse multi-échelle inclut deux homogénéisations :
- la première homogénéisation considère d'une part la matrice formée par les globules de C-S-H solides, et d'autre part les pores dans les phases C-S-H LD et C-S-H HD. Elle permet de calculer les modules d'incompressibilité et de cisaillement des C-S-H LD et C-S-H HD « poreux ». La longueur caractéristique est d'environ $10^{-9}$ - $10^{-8}$m. La matrice occupe 63% de volume pour le cas de la phase C-S-H LD, et elle occupe une proportion supérieure à 76%, dépendant de la température, de volume pour la phase C-S-H HD ;
- la seconde homogénéisation considère d'une part les phases solides (C-S-H LD, C-S-H HD, CH, aluminates et phases initiales réactives du clinker) et d'autre part la porosité capillaire. Elle permet de calculer les modules d'incompressibilité et de cisaillement du système cimentaire et par la suite l'ensemble des paramètres élastiques et hydro-mécaniques. La longueur caractéristique est d'environ $10^{-8}$ m.

**TABLEAU 8**

|  | C$_3$S | C$_2$S | C$_3$A | C$_4$AF | CH | Globule C-S-H |
|---|---|---|---|---|---|---|
| **k [GPa]** | 112,5 | 116,7 | 120,8 | 104,2 | 32,5 | 23,0 |
| **g [GP]** | 51,9 | 53,8 | 55,8 | 48,1 | 14,6 | 17,0 |

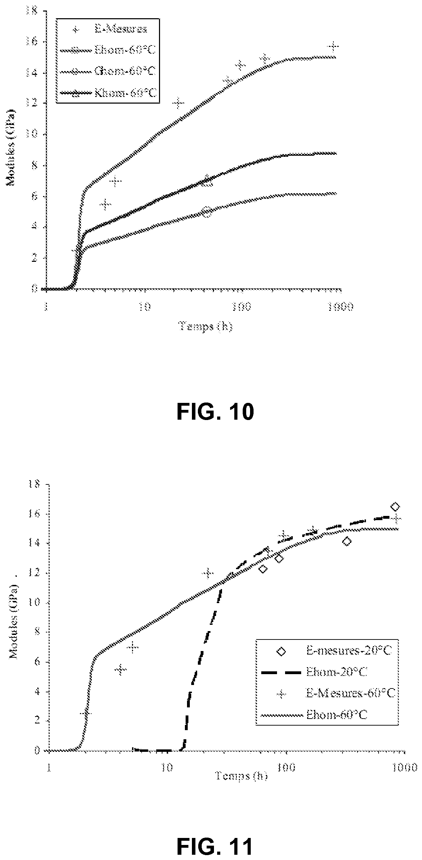

Les figures 10 à 13 montrent une bonne correspondance entre les paramètres mécaniques déterminés selon le procédé de l'invention, et les données expérimentales obtenues dans la littérature (Boumiz et al.[1] ; Haecker et al.[2]), validant ainsi le procédé selon l'invention. Les données issues de la littérature ont été corrigées, afin de transformer les modules dynamiques en modules statiques, à l'aide de la formule suivante :

$$E_{statique} = 0.83 \times E_{dynamique}$$

Sur la figure 10, par exemple, sont comparées les données mesurées (« E-Mesures » dans la légende, symboles en forme de croix) et déterminées par le procédé selon l'invention du module de Young (E), pour une température de cure de 60°C (« Ehom » dans la légende). Les modules d'incompressibilité (K) et de cisaillement (G) déterminés par le procédé selon l'invention sont également représentés (« Khom » et « Ghom » dans la légende). Le module de cisaillement de la pâte de ciment hydratée à 60°C, à l'âge de 1000h (42 jours) est de 6,1GPa. La différence entre cette valeur et la valeur mesurée (5,9GPa) est de 3,3%.

Sur la figure 11, on observe également une bonne correspondance entre les données expérimentales (représentées pas des symboles en croix et en losange, et indiquées en tant que « E-mesures » dans la légende) et les paramètres mécaniques déterminés selon l'invention (courbes en trait plein et en pointillé, et indiqués en tant que « Ehom » dans la légende). Cette figure illustre en outre l'effet de la température d'hydratation sur l'évolution des propriétés élastiques du système cimentaire : la valeur du module de Young à long terme est plus grande pour l'hydratation à 20°C que pour celle à 60°C.

**EP 2 817 615 B1**

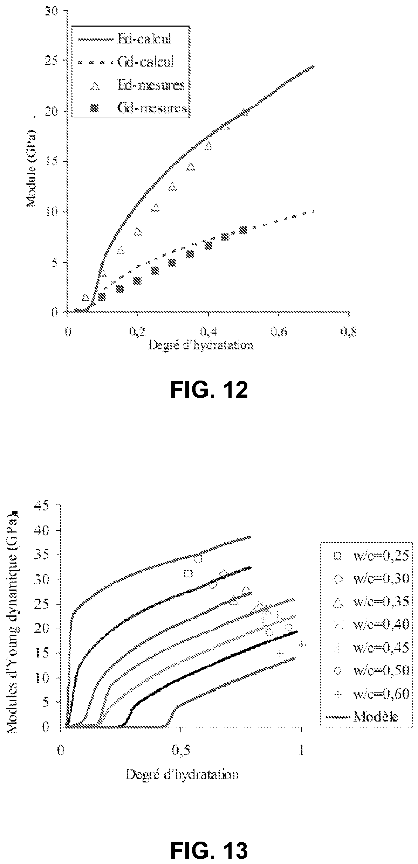

Sur la figure 12, on observe une très bonne correspondance entre les données mesurées (représentées pas des symboles en carré et en triangle, et indiquées en tant que « Ed/Gd-mesures » dans la légende) et prédites selon l'invention (courbes en trait plein et en pointillé, et indiqués en tant que « Ed/Gd-calcul » dans la légende), tant pour le module de Young que pour celui de cisaillement.

Sur la figure 13, on observe également une bonne correspondance entre les données expérimentales (représentées par les différents symboles) et les paramètres mécaniques déterminés selon l'invention (courbes en trait plein). Cette figure illustre en outre l'effet du rapport eau sur ciment sur l'évolution des propriétés élastiques du système cimentaire : la valeur du module de Young à long terme est plus grande pour un rapport eau sur ciment faible.

**Liste de références bibliographiques citées dans la description :**

(1) Boumiz A., Veet C., Cohen Tenoudjit F., "Mechanical properties of cement pastes and mortars at early ages", Advanced Cement based materials, 3, p.94-106 (1996).

(2) Haecker C.J., Garboczi E.J., Bohn J.W., Sun Z., Shah S.P., Voigt T., "Modeling the linear elastic properties of Portland cement paste", Cement concrete research, 35, p. 1948-1960 (2005).

(3) Avrami, M., "Kinetics of phase change, Journal of Chemical Physics", 7, p. 1103-1124, 9, p.177-184 (1939-1940).

(4) Kondo R., Kodama, M., "On the hydration kinetics of cement, Semento Gijutsu Nenpo", 21, p. 77-828 (1967).

(5) Fuji, K., Kondo, W., "Kinetics of the hydration of tricalcium silicate", Journal of the American Ceramic Society, 57 (11), p. 492-497 (1974).

(6) Jennings H. M., Tennis P.D., "Model for the developing microstructure in Portland Cement Pastes", J. Am. Ceram. Soc., 77 (12), p. 3161-3172 (1994).

(7) Reddy, B.R., Santra, A., McMechan, D., Gray, D., Brenneis, C., Dunn, R., "Cement mechanical property measurements under wellbore conditions", SPE 95921 (2005).

(8) Jennings H. M., 'A model for the microstructure of calcium silicate hydrate in cement paste', Cement and Concrete Research, 30, p. 101-116 (2000).

(9) Jennings H. M., "Colloid model of C-S-H and implications to the problem of creep and shrinkage", Materials and Structures/ Concrete Science and Engineering, 37, p. 59-70 (2004).

(10) Constantinides G., "Invariant mechanical properties of Calcium-Silicate-Hydrates (CS-H) in Cement-Based materials: instrumented nanoindentation and microporomechanical modelling", PhD thesis, Massachusetts institute of Technology (2006).

(11) Jennings H. M., "Refinements to colloid model of C-S-H in cement: CM-II", Cement and Concrete Research, 38, p. 275-289 (2008).

(12) Bernard, O., Ulm, F.-J., Lemarchand, E. "A multiscale micromechanics-hydration model for the early-age elastic properties of cement-based materials", Cement and Concrete Research, 33, p. 1293-1309 (2003).

(13) Ghabezloo S., "Association of macroscopic laboratory testing and micromechanics modelling for the evaluation of the poroelastic parameters of a hardened cement paste", Cement and Concrete research, 40 (8), p. 1197-1210 (2010).

(14) Zaoui A., "Continuum micromechanics: survey", Journal of Engineering Mechanics, 128 (8), p. 808-816 (2002).

(15) Rixom, R., Mailvaganam, N. "Chemical admixtures for concrete", Third edition, Spons Architecture Price Book, 456 p. (1999).

(16) Pont Castenada, P., Willis, R. "The effect of spatial distribution on the effective behaviour of composite materials and cracked media", J. Mech. Phys. Solids, 43, p. 1919-1951 (1995).

(17) Hill, R. "The essential structure of constitutive laws for metal composites and polycrystals", J. Mech. Phys. Solids, 15, p. 79-95. (1967).

(18) Eshelby, J.D. "The determination of the elastic field of an ellipsoidal inclusion", Proceedings of the Royal Society of London, 241, p. 376-392 (1957).

(19) Ulm, F.-J., Constantinides, G., Heukamp, F.H. "Is concrete a poromechanics material? - A multiscale investigation of poroelastic properties", Materials and Structures, 37 (265), p. 43-58 (2004).

(20) Ghabezloo S., "Micromechanics analysis of thermal expansion and thermal pressurization of a hardened cement paste", Cement and Concrete research, 41 (5), p. 520-532 (2011).

(21) Bourissai, M. "Comportement thermo-chimio-hydro-mécanique d'un ciment pétrolier au très jeune âge en conditions de prise HP/HT. Approche expérimentale et analyse par changement d'échelle", Thèse de doctorat, Université Paris Est, 246 p. (2010).

**Revendications**

1. Procédé de détermination de paramètres mécaniques d'un système cimentaire de composition initiale $C_0$ et de finesse $\Phi$, en fonction du temps, et en fonction de la finesse du système cimentaire, de la pression et/ou de la température, comprenant les étapes suivantes:

(A) déterminer le degré d'hydratation du système cimentaire en fonction du temps $\alpha(t)$ à partir de la vitesse des ondes de compression en fonction du temps Vp(t) mesurée dans un échantillon du système cimentaire, à une pression $P_1$ et une température $T_1$;

(B) déterminer le degré d'hydratation $\alpha(t)$ en fonction de valeurs souhaitées de finesse $\Phi_n$ du système cimentaire, de pression $P_n$ et/ou de température $T_n$ ;

(C) déterminer la composition du système cimentaire en fonction du temps C(t) et en fonction de valeurs souhaitées de finesse $\Phi_n$ du système cimentaire, de pression $P_n$ et/ou de température $T_n$, à partir du degré d'hydratation $\alpha$ (t) déterminé à l'étape B ;

(D) déterminer au moins un paramètre mécanique du système cimentaire en fonction du temps, et en fonction des valeurs souhaitées de finesse $\Phi_n$ du système cimentaire, de pression $P_n$ et/ou de température $T_n$, à partir de la composition du système cimentaire C(t) déterminée à l'étape C,

dans lequel la finesse du système cimentaire est définie comme étant la finesse de broyage d'un ciment exprimée par sa surface spécifique mesurée à l'aide d'un appareil de type Blaine selon la norme NF EN 196-6, ladite surface spécifique étant définie comme la surface développée par unité de masse.

2. Procédé selon la revendication 1, comprenant en outre une étape initiale de mesure de la vitesse des ondes de compression en fonction du temps $V_p(t)$ dans un échantillon du système cimentaire.

3. Procédé selon la revendication 1 ou 2, dans lequel ledit paramètre mécanique est choisi parmi les paramètres de déformabilité statiques, de préférence les paramètres élastiques statiques, tel que module de Young statique E, le coefficient de Poisson statique v, le module d'incompressibilité K, le module de cisaillement G, et leurs combinaisons.

4. Procédé selon la revendication 3, dans lequel deux paramètres élastiques statiques sont déterminés.

5. Procédé selon la revendication 3 ou 4, comprenant en outre la détermination d'un paramètre mécanique choisi parmi les paramètres de couplage hydro-mécanique, tel que le coefficient de Biot ou le coefficient de Skempton, et leurs combinaisons.

6. Procédé selon l'une des revendications précédentes, dans lequel le degré d'hydratation du système cimentaire en fonction du temps $\alpha(t)$ est calculé à partir de $V_p(t)$ selon une relation linéaire.

7. Procédé selon la revendication 6, dans lequel le degré d'hydratation du système cimentaire en fonction du temps $\alpha(t)$ est calculé à partir de $V_p(t)$ selon la relation $\alpha = (V_P - V_0)/(V\infty - V_0)$, avec $V_0$ et $V_P$ correspondant respectivement à la vitesse des ondes de compression mesurée dans l'échantillon du système cimentaire au temps $t = 0$ et au temps t, et $V_\infty$ correspondant à la vitesse des ondes de compression dans l'échantillon du système cimentaire complètement hydraté.

8. Procédé selon l'une des revendications précédentes, dans lequel le processus d'hydratation comprend un premier stade où l'hydratation est gouvernée principalement par un processus de nucléation et de croissance, et un deuxième stade où l'hydratation est gouvernée principalement par un processus diffusion des ions, ledit deuxième stade commençant lorsque le degré d'hydratation $\alpha$ atteint une valeur de degré d'hydratation seuil $\alpha^*$, cette valeur seuil $\alpha^*$ étant fonction de la température, et dans lequel l'étape B comprend les sous-étapes suivantes :

(B-i) la détermination du degré d'hydratation $\alpha(t)$ pendant le premier stade du processus d'hydratation du système cimentaire ;

(B-ii) la détermination du degré d'hydratation $\alpha(t)$ pendant le deuxième stade du processus d'hydratation du système cimentaire ;

chacune des sous-étapes B-i et B-ii prenant en compte la finesse $\Phi$ du système cimentaire, la pression et la température pour la détermination du degré d'hydratation $\alpha(t)$.

**9.** Procédé selon la revendication 8, dans lequel la valeur de degré d'hydratation seuil $\alpha^*$ est évaluée en minimisant l'écart entre $\alpha(t)$ déterminé en utilisant le modèle cinétique 120 et $\alpha(t)$ déterminé expérimentalement à partir de la vitesse des ondes de compression, pour différentes températures, et à une pression constante, de manière à prendre en compte la variation de $\alpha^*$ en fonction de la température dans l'étape B.

**10.** Procédé selon la revendication 9, dans lequel :

- le système cimentaire de composition initiale $C_0$ comprend un ciment et de l'eau, le ciment comprenant au moins une phase initiale réactive X ;
- le degré d'hydratation $\alpha(t)$ déterminé à l'étape B correspond à la moyenne pondérée des degrés d'hydratation de chacune des phases initiales réactives X du ciment ;
- le degré d'hydratation de chacune des phases initiales réactives X du ciment est fonction du rapport entre l'affinité chimique $A_x(\alpha)$ de la phase initiale réactive X, cette affinité chimique $A_x(\alpha)$ contrôlant le taux d'évolution de l'hydratation de la phase initiale réactive X, et le temps caractéristique associé à la réaction de la phase initiale réactive X avec l'eau $\tau_x$ ;
- et le temps caractéristique associé à la réaction de la phase initiale réactive X avec l'eau $\tau_x$ est fonction de la finesse $\Phi$ du ciment, de la pression et de la température.

**11.** Procédé selon la revendication 10, dans lequel le temps caractéristique associé à la réaction de la phase initiale réactive X avec l'eau $\tau_X$ s'exprime selon l'équation suivante :

$$\tau_x\left(T,\Phi\right) + \sqrt[n_x+1]{\frac{\Phi_0}{\Phi}} \times \tau_x\left(T_0,\Phi_0\right)\exp\left(\frac{\Delta E_x}{R}\left(\frac{1}{T}-\frac{1}{T_0}\right)+\frac{\Delta V_x}{R}\left(\frac{P}{T}-\frac{P_0}{T_0}\right)\right) \qquad (VI)$$

avec $\Phi_0$ la finesse d'un ciment de référence, de préférence $\Phi_0 = 3600$ cm$^2$/g, $T_0$ la température initiale et $P_0$ la pression initiales au temps t=0 du processus d'hydratation, R la constante de gaz parfaits, $\Delta E_x$ l'énergie d'activation, $\Delta V_x$ le volume d'activation pour la nucléation et la croissance des hydrates lors du premier stade du processus d'hydratation et $n_x$ une constante.

**12.** Procédé selon l'une des revendications précédentes, dans lequel le système cimentaire comprend *m* phases, parmi lesquelles :

- au moins une phase initiale réactive X, de préférence choisie dans le groupe constitué par le silicate tricalcique $C_3S$, le silicate dicalcique $C_2S$, l'aluminate tricalcique $C_3A$, l'aluminoferrite tétracalcique $C_4F$, et leurs associations;
- au moins une phase hydratée Y issue de l'hydratation d'au moins une phase initiale réactive X, de préférence choisie dans le groupe constitué par le silicate de calcium hydraté C-S-H, l'hydroxyde de calcium CH, le trisulfoaluminate de calcium hydraté TSA, le monosulfate de calcium hydraté AFm, l'aluminoferrite de calcium hydraté, et leurs associations ;
- de l'eau;

la composition initiale du système cimentaire $C_0$ comprenant un volume initial d'eau déterminé $V_w^0$ et au moins une phase initiale réactive X,
et dans lequel l'étape C comprend l'estimation de la composition du système cimentaire en fonction du temps C(t) par la détermination de la fraction molaire, massique ou volumique des m phases du système cimentaire.

**13.** Procédé selon la revendication 12, dans lequel :

- le volume d'au moins une phase initiale réactive X en fonction du temps $V_x(t)$ est calculé selon l'équation *(XIV)* :

$$V_x\left(t\right) = V_x^0\left(1-\alpha_x\left(t\right)\right) \qquad (XIV)$$

avec $V_x^0$ le volume initial de la phase initiale réactive X, et $\alpha_x(t)$ le degré d'hydratation de la phase initiale réactive X en fonction du temps ;
- le volume d'au moins une phase hydratée en fonction du temps est calculé selon l'équation *(XV)* :

$$V(t) = \sum V_y^x \cdot \alpha_x(t) \qquad (XV)$$

avec $V_y^x$ le volume occupé par la phase hydratée *Y* formée par la phase réactive *X* dans un volume élémentaire représentatif, et $\alpha_x(t)$ le degré d'hydratation de la phase réactive *X* en fonction du temps ;
- le volume de l'eau en fonction du temps $V_w(t)$ est calculé selon l'équation *(XVI)* :

$$V_w(t) = V_w^0 - \sum V_w^x \cdot \alpha_x(t) \qquad (XVI)$$

avec $V_w^0$ le volume initial d'eau dans le système cimentaire, $V_w^x$ le volume de l'eau consommée par la phase X, $\alpha_x(t)$ le degré d'hydratation de X en fonction du temps ;

**14.** Procédé selon l'une des revendications précédentes, dans lequel l'étape D est réalisée selon une méthode d'analyse multi-compositionnelle (140), cette méthode d'analyse multi-compositionnelle (140) :

- prenant en compte un modèle multi-échelles du système cimentaire, ledit modèle multi-échelles comprenant au moins une échelle élémentaire représentant des globules de la phase solide C-S-H et une échelle macroscopique du système cimentaire, de préférence un modèle multi-échelles comprenant trois échelles ;
- et permettant la détermination d'au moins un paramètre mécanique du système cimentaire en fonction du temps, pour une valeur donnée de finesse du système cimentaire, de pression et/ou de température, en utilisant des techniques d'homogénéisation, de préférence le schéma de Mori-Tanaka et le schéma auto-cohérent, connaissant la composition du système cimentaire C(t) déterminée à l'étape C, et connaissant l'évolution des composants du ciment du système cimentaire obtenue à partir du modèle de la cinétique d'hydratation utilisé à l'étape A.

**15.** Procédé selon l'une des revendications précédentes, dans lequel le système cimentaire comprend du ciment Portland.

**16.** Procédé selon l'une des revendications précédentes, permettant de caractériser le comportement mécanique d'un système cimentaire utilisé en tant que gaine ou bouchon de ciment dans un puits, de préférence un puits pétrolier.

**17.** Produit programme informatique permettant de conserver dans une mémoire d'une unité d'un processeur ou sur un support de mémoire amovible approprié pour coopérer avec ladite unité du processeur, le produit-programme comprenant des instructions pour mettre en oeuvre le procédé selon l'une quelconque de revendications 1 à 16.

**Patentansprüche**

**1.** Verfahren zum Bestimmen von mechanischen Parametern eines Zementsystems der ursprünglichen Zusammensetzung $C_0$ und der Feinheit $\phi$ als Funktion der Zeit und als Funktion der Feinheit des Zementsystems, des Drucks und/oder der Temperatur, umfassend die folgenden Schritte:

(A) Bestimmen des Hydrationsgrads des Zementsystems als Funktion der Zeit $\alpha(t)$ ausgehend von der Geschwindigkeit von Kompressionswellen als Funktion der Zeit $V_p(t)$, gemessen in einer Probe des Zementsystems bei einem Druck $P_1$ und einer Temperatur $T_1$;
(B) Bestimmen des Hydrationsgrads $\alpha(t)$ als Funktion von gewünschten Werten der Feinheit $\phi_n$ des Zementsystems, des Drucks $P_n$ und/oder der Temperatur $T_n$;
(C) Bestimmen der Zusammensetzung des Zementsystems als Funktion der Zeit $C(t)$ und als Funktion von gewünschten Werten der Feinheit $\phi_n$ des Zementsystems, des Drucks $P_n$ und/oder der Temperatur $T_n$, ausgehend von dem im Schritt B bestimmten Hydrationsgrad $\alpha(t)$;
(D) Bestimmen von wenigstens einem mechanischen Parameter des Zementsystems als Funktion der Zeit und als Funktion der gewünschten Werte der Feinheit $\phi_n$ des Zementsystems, des Drucks $P_n$ und/oder der Tem-

peratur $T_n$, ausgehend von der in Schritt C bestimmten Zusammensetzung des Zementsystems C(t), wobei die Feinheit des Zementsystems als die Feinheit einer Zerkleinerung eines Zement definiert ist, ausgedrückt durch seine spezifische Oberfläche, welche mit Hilfe einer Vorrichtung vom Blaine-Typ nach der Norm NF EN 196-6 gemessen wird, wobei die spezifische Oberfläche als die entstehende Oberfläche pro Masseneinheit definiert ist.

2. Verfahren nach Anspruch 1, ferner umfassend einen Ausgangsschritt eines Messens der Geschwindigkeit von Kompressionswellen als Funktion der Zeit $V_p$(t) in einer Probe des Zementsystems.

3. Verfahren nach Anspruch 1 oder 2, wobei der mechanische Parameter aus den Parametern der statischen Verformbarkeit ausgewählt ist, insbesondere den statisch-elastischen Parametern, wie beispielsweise dem statischen Young-Modul E, dem statischen Poisson-Koeffizienten v, dem Inkompressibilitätsmodul K, dem Schermodul G, und ihren Kombinationen.

4. Verfahren nach Anspruch 3, wobei zwei statisch-elastische Parameter bestimmt werden.

5. Verfahren nach Anspruch 3 oder 4, ferner umfassend das Bestimmen eines mechanischen Parameters, welcher ausgewählt ist aus den Parametern der hydromechanischen Kopplung, wie beispielsweise dem Biot-Koeffizienten oder dem Skempton-Koeffizienten, und ihren Kombinationen.

6. Verfahren nach einem der vorhergehenden Ansprüche, wobei der Hydrationsgrad des Zementsystems als Funktion der Zeit $\alpha$(t) ausgehend von $V_p$(t) gemäß einer linearen Beziehung berechnet wird.

7. Verfahren nach Anspruch 6, wobei der Hydrationsgrad des Zementsystems als Funktion der Zeit $\alpha$(t) ausgehend von $V_p$(t) gemäß der Beziehung $\alpha = (V_p - V_0)/(V_\infty - V_0)$ berechnet wird, wobei $V_0$ und $V_p$ der Geschwindigkeit von Kompressionswellen gemessen in der Probe des Zementsystems zur Zeit t = 0 bzw. zur Zeit t entsprechen, und wobei $V_\infty$ der Geschwindigkeit von Kompressionswellen in der Probe des vollständig hydrierten Zementsystems entspricht.

8. Verfahren nach einem der vorhergehenden Ansprüche, wobei der Hydrationsprozess eine erste Stufe, in welcher die Hydration hauptsächlich durch einen Keimbildungs- und Wachstumsprozess gelenkt wird, und eine zweite Stufe umfasst, in welcher die Hydration hauptsächlich durch einen Ionen-Diffusionsprozess gelenkt wird, wobei die zweite Stufe beginnt, wenn der Hydrationsgrad $\alpha$ einen Hydrationsgrad-Schwellenwert $\alpha^*$ erreicht, wobei der Schwellenwert $\alpha^*$ eine Funktion der Temperatur ist, und wobei der Schritt B die folgenden Unterschritte umfasst:

(B-i) die Bestimmung des Hydrationsgrads $\alpha$(t) während der ersten Stufe des Hydrationsprozesses des Zementsystems;
(B-ii) die Bestimmung des Hydrationsgrads $\alpha$(t) während der zweiten Stufe des Hydrationsprozesses des Zementsystems;

wobei jeder der Unterschritte B-i und B-ii die Feinheit $\Phi$ des Zementsystems, den Druck und die Temperatur für die Bestimmung des Hydrationsgrads $\alpha$(t) berücksichtigt.

9. Verfahren nach Anspruch 8, wobei der Hydrationsgrad-Schwellenwert $\alpha^*$ ausgewertet wird, indem die Differenz zwischen $\alpha$(t), welches unter Verwendung des kinetischen Modells 120 bestimmt wird, und $\alpha$(t), welches experimentell ausgehend von der Geschwindigkeit von Kompressionswellen bestimmt wird, minimiert wird, für verschiedene Temperaturen und bei einem konstanten Druck, indem die Variation von $\alpha^*$ als Funktion der Temperatur in Schritt B berücksichtigt wird.

10. Verfahren nach Anspruch 9, wobei:

- das Zementsystem der Ausgangszusammensetzung $C_0$ einen Zement und Wasser umfasst, wobei der Zement wenigstens eine reaktive Ausgangsphase X umfasst;
- der Hydrationsgrad $\alpha$(t), welcher in Schritt B bestimmt wird, dem gewichteten Durchschnitt von Hydrationsgraden von jeder der reaktiven Ausgangsphasen X des Zements entspricht;
- der Hydrationsgrad von jeder der reaktiven Ausgangsphasen X des Zements eine Funktion des Verhältnisses zwischen der chemischen Affinität $A_x(\alpha)$ der reaktiven Ausgangsphase X, wobei die chemische Affinität $A_x(\alpha)$ die Rate des Fortschreitens der Hydration der reaktiven Ausgangsphase X steuert, und der charakteristischen Zeit ist, welche der Reaktion der reaktiven Ausgangsphase X mit dem Wasser $T_x$ zugeordnet ist;

- und die charakteristische Zeit, welche der Reaktion der reaktiven Ausgangsphase X mit dem Wasser $T_x$ zugeordnet ist, eine Funktion der Feinheit $\Phi$ des Zements, des Drucks und der Temperatur ist.

11. Verfahren nach Anspruch 10, wobei die charakteristische Zeit, welche der Reaktion der reaktiven Ausgangsphase X mit dem Wasser $T_x$ zugeordnet ist, durch die folgende Gleichung ausgedrückt wird:

$$\tau_x(T,\Phi) + {}^{n_x+1}\sqrt{\frac{\Phi_0}{\Phi}} \times \tau_x(T_0,\Phi_0)\exp\left(\frac{\Delta E_x}{R}\left(\frac{1}{T}-\frac{1}{T_0}\right)+\frac{\Delta V_x}{R}\left(\frac{P}{T}-\frac{P_0}{T_0}\right)\right) \quad \text{(VI)}$$

wobei $\Phi_0$ die Feinheit eines Referenzzements ist, vorzugsweise $\Phi_0 = 3600$ cm$^2$/g, $T_0$ die Ausgangstemperatur und $P_0$ der Ausgangsdruck zur Zeit t=0 des Hydrationsprozesses sind, R die ideale Gaskonstante ist, $\Delta E_x$ die Aktivierungsenergie ist, $\Delta V_x$ das Aktivierungsvolumen für die Keimbildung und das Wachsen der Hydrate während der ersten Stufe des Hydrationsprozesses ist und $n_x$ eine Konstante ist.

12. Verfahren nach einem der vorhergehenden Ansprüche, wobei das Zementsystem m Phasen umfasst, einschließlich:

- wenigstens eine reaktive Ausgangsphase X, vorzugsweise ausgewählt aus der Gruppe, bestehend aus Tricalciumsilikat $C_3S$, Dicalciumsilikat $C_2S$, Tricalciumaluminat $C_3A$, Tetracalcium-Aluminoferrit $C_4F$ und Kombinationen daraus;
- wenigstens eine hydrierte Phase Y, entstanden aus der Hydration von wenigstens einer reaktiven Ausgangsphase X, vorzugsweise ausgewählt aus der Gruppe, gebildet aus hydriertem Calciumsilikat C-S-H, Calciumhydroxid CH, hydriertem Calciumtrisulfoaluminat TSA, hydriertem Calciummonosulfat AFm, hydriertem Calciumaluminoferrit und Kombinationen daraus;
- Wasser;

wobei die Ausgangszusammensetzung des Zementsystems $C_0$ ein vorbestimmtes Ausgangsvolumen von Wasser $V_w^0$ und wenigstens eine reaktive Ausgangsphase X umfasst,
und wobei der Schritt C das Schätzen der Zusammensetzung des Zementsystems als Funktion der Zeit C(t) für die Bestimmung des molaren, Massen- oder Volumenanteils der m Phasen des Zementsystems umfasst.

13. Verfahren nach Anspruch 12, wobei:

- das Volumen der wenigstens einen reaktiven Ausgangsphase X als Funktion der Zeit $V_x(t)$ gemäß Gleichung (XIV) berechnet wird:

$$V_x(t) = V_x^0\big(1-\alpha_x(t)\big) \quad \text{(XIV)}$$

wobei $V_x^0$ das Ausgangsvolumen der reaktiven Ausgangsphase X ist und $\alpha_x(t)$ der Hydrationsgrad der reaktiven Ausgangsphase X als Funktion der Zeit ist;
- das Volumen der wenigstens einen hydrierten Phase als Funktion der Zeit nach Gleichung (XV) berechnet wird:

$$V(t) = \sum V_y^x \cdot \alpha_x(t) \quad \text{(XV)}$$

wobei $V_y^x$ das durch die hydrierte Phase Y eingenommene Volumen ist, welches durch die reaktive Phase X in einem repräsentativen Elementarvolumen gebildet wird, und $\alpha_x(t)$ der Hydrationsgrad der reaktiven Phase X als Funktion der Zeit ist;
- das Volumen des Wasser als Funktion der Zeit $V_w(t)$ gemäß Gleichung (XVI) berechnet wird:

$$V_w(t) = V_w^0 - \sum V_w^x \cdot \alpha_x(t) \quad \text{(XVI)}$$

wobei $V_w^0$ das Ausgangsvolumen des Wassers in dem Zementsystem ist, $V_w^x$ das Volumen des durch die Phase X verbrauchten Wassers ist, $\alpha_x(t)$ der Hydrationsgrad von X als Funktion der Zeit ist.

14. Verfahren nach einem der vorhergehenden Ansprüche, wobei der Schritt D gemäß eines Mehrkomponenten-Analyseverfahrens (140) durchgeführt wird, wobei das Mehrkomponenten-Analyseverfahren (140) umfasst:

- Berücksichtigen eines mehrskaligen Modells des Zementsystems, wobei das mehrskalige Modell wenigstens eine Elementarskala, welche Kügelchen der festen Phase C-S-H repräsentiert, sowie eine makroskopische Skala des Zementsystems umfasst, vorzugsweise eines mehrskaligen Modells, welches drei Skalen umfasst;
- und Erlauben des Bestimmens von wenigstens einem mechanischen Parameter des Zementsystems als Funktion der Zeit für einen gegebenen Wert einer Feinheit des Zementsystems, des Drucks und/oder der Temperatur, unter Verwendung von Techniken zur Homogenisierung, vorzugsweise dem Mori-Tanaka-Schema und dem Autokohärenz-Schema, wobei die bei Schritt C bestimmte Zusammensetzung des Zementsystems C(t) bekannt ist, und die Entwicklung der Komponenten des Zements des Zementsystems, welche ausgehend von dem Modell der Kinetik der Hydration erhalten wird, welches in Schritt A verwendet wird, bekannt ist.

15. Verfahren nach einem der vorhergehenden Ansprüche, wobei das Zementsystem Portland-Zement umfasst.

16. Verfahren nach einem der vorhergehenden Ansprüche, wobei ein Charakterisieren des mechanischen Verhaltens eines Zementsystems erlaubt wird, welches als eine Hülle oder ein Pfropfen aus Zement für eine Quelle verwendet wird, vorzugsweise für eine Ölquelle.

17. Computerprogramm-Produkt, welches geeignet ist, in einem Speicher einer Prozessoreinheit oder auf einem geeigneten entfernbaren Speichermedium zum Zusammenwirken mit der Prozessoreinheit gespeichert zu sein, wobei das Programm-Produkt Anweisungen umfasst, um das Verfahren nach einem der Ansprüche 1 bis 16 auszuführen.

**Claims**

1. A method for determining mechanical parameters of a cement system of initial composition $C_0$ and of fineness $\Phi$, as a function of time, and as a function of the fineness of the cement system, pressure and/or temperature, comprising the following steps:

(A) determining the degree of hydration of the cement system as a function of time $\alpha(t)$ from the velocity of the compression waves as a function of time $V_p(t)$ measured in a specimen of the cement system, at a pressure $P_1$ and a temperature $T_1$;
(B) determining the degree of hydration $\alpha(t)$ as a function of desired values of fineness $\Phi_n$ of the cement system, of pressure $P_n$ and/or of temperature $T_n$;
(C) determining the composition of the cement system as a function of time $C(t)$ and as a function of desired values of fineness $\Phi_n$ of the cement system, of pressure $P_n$ and/or of temperature $T_n$, from the degree of hydration $\alpha(t)$ determined in step B;
(D) determining at least one mechanical parameter of the cement system as a function of time, and as a function of the desired values of fineness $\Phi_n$ of the cement system, of pressure $P_n$ and/or of temperature $T_n$, from the composition of the cement system $C(t)$ determined in step C;

wherein the fineness of the cement system is defined as the fineness of grinding of a cement expressed by its specific surface measured using apparatus of the Blaine type according to standard NF EN 196-6, said specific surface being defined as the developed surface per unit mass.

2. The method as claimed in claim 1, further comprising an initial step of measurement of the velocity of the compression waves as a function of time $V_p(t)$ in a specimen of the cement system.

3. The method as claimed in claim 1 or 2, wherein said mechanical parameter is selected from the static deformability parameters, preferably the static elastic parameters, such as static Young's modulus E, static Poisson's ratio v, bulk modulus K, shear modulus G, and combinations thereof.

4. The method as claimed in claim 3, wherein two static elastic parameters are determined.

5. The method as claimed in claim 3 or 4, further comprising determination of a mechanical parameter selected from the parameters of hydro-mechanical coupling, such as Biot's coefficient or Skempton's coefficient, and combinations thereof.

6. The method as claimed in one of the preceding claims, wherein the degree of hydration of the cement system as a function of time $\alpha(t)$ is calculated from Vp(t) according to a linear relation.

7. The method as claimed in claim 6, wherein the degree of hydration of the cement system as a function of time $\alpha(t)$ is calculated from $V_p(t)$ according to the relation $\alpha = (V_P - V_0)/(V_\infty - V_0)$, with $V_0$ and $V_P$ corresponding respectively to the velocity of the compression waves measured in the specimen of the cement system at time $t = 0$ and at time t, and $V_\infty$ corresponding to the velocity of the compression waves in the specimen of the fully hydrated cement system.

8. The method as claimed in one of the preceding claims, wherein the hydration process comprises a first stage in which hydration is mainly governed by a process of nucleation and growth, and a second stage in which hydration is mainly governed by an ion diffusion process, said second stage starting when the degree of hydration $\alpha$ reaches a threshold value degree of hydration $\alpha^*$, this threshold value $\alpha^*$ being a function of the temperature, and in which step B comprises the following substeps:

   (B-i) determination of the degree of hydration $\alpha(t)$ during the first stage of the process of hydration of the cement system;
   (B-ii) determination of the degree of hydration $\alpha(t)$ during the second stage of the process of hydration of the cement system;

   each of the substeps B-i and B-ii taking into account the fineness $\Phi$ of the cement system, the pressure and the temperature for determining the degree of hydration $\alpha(t)$.

9. The method as claimed in claim 8, wherein the threshold value of degree of hydration $\alpha^*$ is evaluated by minimizing the difference between $\alpha(t)$ determined using the kinetic model 120 and $\alpha(t)$ determined experimentally from the velocity of the compression waves, for different temperatures, and at a constant pressure, so as to take into account the variation of $\alpha^*$ as a function of the temperature in step B.

10. The method as claimed in claim 9, wherein:

   - the cement system of initial composition $C_0$ comprises a cement and water, the cement comprising at least one reactive initial phase X;
   - the degree of hydration $\alpha(t)$ determined in step B corresponds to the weighted average of the degrees of hydration of each of the reactive initial phases X of the cement;
   - the degree of hydration of each of the reactive initial phases X of the cement is a function of the ratio of the chemical affinity $A_x(\alpha)$ of the reactive initial phase X, this chemical affinity $A_x(\alpha)$ controlling the rate of variation of the hydration of the reactive initial phase X, to the characteristic time associated with reaction of the reactive initial phase X with water $\tau_x$;
   - and the characteristic time associated with reaction of the reactive initial phase X with water $\tau_x$ is a function of the fineness $\Phi$ of the cement, the pressure and the temperature.

11. The method as claimed in claim 10, wherein the characteristic time associated with reaction of the reactive initial phase X with water $\tau_X$ is expressed according to the following equation:

$$\tau_x(T,\Phi) + \sqrt[n_x+1]{\frac{\Phi_0}{\Phi}} \times \tau_x(T_0,\Phi_0) \exp\left(\frac{\Delta E_x}{R}\left(\frac{1}{T} - \frac{1}{T_0}\right) + \frac{\Delta V_x}{R}\left(\frac{P}{T} - \frac{P_0}{T_0}\right)\right) \qquad (VI)$$

where $\Phi_0$ is the fineness of a reference cement, preferably $\Phi_0 = 3600$ cm$^2$/g, $T_0$ is the initial temperature and $P_0$ is the initial pressure at time t=0 of the hydration process, R is the gas constant, $\Delta E_x$ is the activation energy, $\Delta V_x$ is the activation volume for nucleation and growth of the hydrates during the first stage of the hydration process and $n_x$ is a constant.

12. The method as claimed in one of the preceding claims, wherein the cement system comprises *m* phases, including:

   - at least one reactive initial phase X, preferably selected from the group consisting of tricalcium silicate $C_3S$, dicalcium silicate $C_2S$, tricalcium aluminate $C_3A$, tetracalcium aluminoferrite $C_4F$, and combinations thereof;
   - at least one hydrated phase Y resulting from the hydration of at least one reactive initial phase X, preferably selected from the group consisting of hydrated calcium silicate C-S-H, calcium hydroxide CH, hydrated calcium trisulfoaluminate TSA, hydrated calcium monosulfate AFm, hydrated calcium aluminoferrite, and combinations thereof;
- water;

the initial composition of the cement system $C_0$ comprising a defined initial volume of water $V_w^0$ and at least one reactive initial phase X,
and in which step C comprises estimation of the composition of the cement system as a function of time C(t) by determining the mole, mass or volume fraction of the m phases of the cement system.

13. The method as claimed in claim 12, wherein:

- the volume of at least one reactive initial phase X as a function of time $V_x(t)$ is calculated according to equation *(XIV)*:

$$V_x(t) = V_x^0 \left(1 - \alpha_x(t)\right) \qquad (XIV)$$

with $V_x^0$ the initial volume of the reactive initial phase X, and $\alpha_x(t)$ the degree of hydration of the reactive initial phase X as a function of time;
- the volume of at least one hydrated phase as a function of time is calculated according to equation *(XV)*:

$$V(t) = \sum V_y^x \cdot \alpha_x(t) \qquad (XV)$$

where $V_y^x$ is the volume occupied by the hydrated phase *Y* formed by the reactive phase *X* in a representative volume element, and $\alpha_x(t)$ is the degree of hydration of the reactive phase *X* as a function of time;
- the volume of water as a function of time $V_w(t)$ is calculated according to equation *(XVI)*:

$$V_w(t) = V_w^0 - \sum V_w^x \cdot \alpha_x(t) \qquad (XVI)$$

where $V_w^0$ is the initial volume of water in the cement system, $V_w^x$ is the volume of water consumed by phase X, $\alpha_x(t)$ is the degree of hydration of X as a function of time.

14. The method as claimed in one of the preceding claims, wherein step D is carried out according to a method of multi-compositional analysis (140), said method of multi-compositional analysis (140):

- taking into account a multi-scale model of the cement system, said multi-scale model comprising at least one elementary scale representing globules of the solid phase C-S-H and a macroscopic scale of the cement system, preferably a multi-scale model comprising three scales;
- and allowing determination of at least one mechanical parameter of the cement system as a function of time, for a given value of fineness of the cement system, of pressure and/or of temperature, using homogenization techniques, preferably the Mori-Tanaka scheme and the self-consistent scheme, knowing the composition of the cement system C(t) determined in step C, and knowing the evolution of the components of the cement of the cement system obtained from the model of the hydration kinetics used in step A.

15. The method as claimed in one of the preceding claims, wherein the cement system comprises Portland cement.

16. The method as claimed in one of the preceding claims, for characterizing the mechanical behavior of a cement system used as cement sheath or plug in a well, preferably an oil well.

**17.** A software product for storing, in a memory of a unit of a processor or on a removable storage medium suitable for interacting with said processor unit, the software product comprising instructions for carrying out the method as claimed in any one of claims 1 to 16.

Cem $C_0$, $V_p(t)$

— 110

A

$\alpha(t)$, $P_1$, $T_1$

— 120

B

$\alpha(t)$, $P_n$, $T_n$

— 130

C

$C(t)$, $P_n$, $T_n$

— 140

D

Para =
$E(t)$, $v(t)$, ...

**FIG. 1**

**FIG. 2**

**FIG. 3** (ART ANTERIEUR)

**FIG. 4**

$$V = 2500\alpha + 1479$$

**FIG. 5**

FIG. 6

FIG. 7

**FIG. 8**

**FIG. 9**

**FIG. 10**

**FIG. 11**

**FIG. 12**

**FIG. 13**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1541987 A
- US 7621186 B
- WO 2007020435 A
- US 7089816 B
- US 7549320 B
- US 7552648 B
- US 4259868 A
- US 5859811 A
- US 5763773 A
- US 5357481 A
- US 5168470 A
- US 5001676 A
- US 4813028 A
- US 4779236 A
- US 4255798 A
- US 3401773 A
- US 2538114 A

**Littérature non-brevet citée dans la description**

- **BOUMIZ A. ; VEET C. ; COHEN TENOUDJIT F.** Mechanical properties of cement pastes and mortars at early ages. *Advanced Cement based materials,* 1996, vol. 3, 94-106
- **HAECKER C.J. ; GARBOCZI E.J. ; BOHN J.W. ; SUN Z. ; SHAH S.P. ; VOIGT T.** Modeling the linear elastic properties of Portland cement paste. *Cement concrete research,* 2005, vol. 35, 1948-1960
- **AVRAMI, M.** Kinetics of phase change. *Journal of Chemical Physics,* vol. 7, 1103-1124
- *JOURNAL OF CHEMICAL PHYSICS,* 1939, vol. 9, 177-184
- **KONDO R. ; KODAMA, M.** On the hydration kinetics of cement. *Semento Gijutsu Nenpo,* 1967, vol. 21, 77-828
- **FUJI, K. ; KONDO, W.** Kinetics of the hydration of tricalcium silicate. *Journal of the American Ceramic Society,* 1974, vol. 57 (11), 492-497
- **JENNINGS H. M. ; TENNIS P.D.** Model for the developing microstructure in Portland Cement Pastes. *J. Am. Ceram. Soc.,* 1994, vol. 77 (12), 3161-3172
- **REDDY, B.R. ; SANTRA, A. ; MCMECHAN, D. ; GRAY, D. ; BRENNEIS, C. ; DUNN, R.** Cement mechanical property measurements under wellbore conditions. *SPE,* 2005, 95921
- **JENNINGS H. M.** A model for the microstructure of calcium silicate hydrate in cement paste. *Cement and Concrete Research,* 2000, vol. 30, 101-116
- **JENNINGS H. M.** Colloid model of C-S-H and implications to the problem of creep and shrinkage. *Materials and Structures/ Concrete Science and Engineering,* 2004, vol. 37, 59-70
- Invariant mechanical properties of Calcium-Silicate-Hydrates (CS-H) in Cement-Based materials: instrumented nanoindentation and microporomechanical modelling. **CONSTANTINIDES G.** PhD thesis. Massachusetts institute of Technology, 2006
- **JENNINGS H. M.** Refinements to colloid model of C-S-H in cement: CM-II. *Cement and Concrete Research,* 2008, vol. 38, 275-289
- **BERNARD, O. ; ULM, F.-J. ; LEMARCHAND, E.** A multiscale micromechanics-hydration model for the early-age elastic properties of cement-based materials. *Cement and Concrete Research,* 2003, vol. 33, 1293-1309
- **GHABEZLOO S.** Association of macroscopic laboratory testing and micromechanics modelling for the evaluation of the poroelastic parameters of a hardened cement paste. *Cement and Concrete research,* 2010, vol. 40 (8), 1197-1210
- **ZAOUI A.** Continuum micromechanics: survey. *Journal of Engineering Mechanics,* 2002, vol. 128 (8), 808-816
- **RIXOM, R. ; MAILVAGANAM, N.** Chemical admixtures for concrete. Spons Architecture Price Book, 1999, 456
- **PONT CASTENADA, P. ; WILLIS, R.** The effect of spatial distribution on the effective behaviour of composite materials and cracked media. *J. Mech. Phys. Solids,* 1995, vol. 43, 1919-1951
- **HILL, R.** The essential structure of constitutive laws for metal composites and polycrystals. *J. Mech. Phys. Solids,* 1967, vol. 15, 79-95
- **ESHELBY, J.D.** The determination of the elastic field of an ellipsoidal inclusion. *Proceedings of the Royal Society of London,* 1957, vol. 241, 376-392

- **ULM, F.-J. ; CONSTANTINIDES, G. ; HEUKAMP, F.H.** Is concrete a poromechanics material? - A multiscale investigation of poroelastic properties. *Materials and Structures,* 2004, vol. 37 (265), 43-58
- **GHABEZLOO S.** Micromechanics analysis of thermal expansion and thermal pressurization of a hardened cement paste. *Cement and Concrete research,* 2011, vol. 41 (5), 520-532
- Comportement thermo-chimio-hydro-mécanique d'un ciment pétrolier au très jeune âge en conditions de prise HP/HT. Approche expérimentale et analyse par changement d'échelle. **BOURISSAI, M.** Thèse de doctorat. Université Paris Est, 2010, 246